(19) Europäisches Patentamt
European Patent Office
Office européen des brevets

(11) Publication number: 0 145 303 A1

# (12) EUROPEAN PATENT APPLICATION

(21) Application number: 84307817.1

(22) Date of filing: 12.11.84

(51) Int. Cl.⁴: C 07 C 125/063
C 07 C 93/00, C 07 C 117/00
C 07 C 127/15, A 61 K 31/13
A 61 K 31/16, A 61 K 31/17
A 61 K 31/22, A 61 K 31/27

(30) Priority: 14.11.83 JP 212514/83

(43) Date of publication of application: 19.06.85 Bulletin 85/25

(84) Designated Contracting States: AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Arai, Yoshinobu
1-5-8-505, Wakayama-dai Shimamoto-cho
Mishima-gun Osaka(JP)

(72) Inventor: Hamanaka, Nobuyuki
11-38 Hiroshiki Ohyamazaki-cho
Otokuni-gun Kyoto(JP)

(72) Inventor: Miyamoto, Tsumoru
1-40, Tonomoriyama
Jyouyou-shi Kyoto(JP)

(74) Representative: Pearce, Anthony Richmond et al,
Marks & Clerk Alpha Tower Suffolk Street
Queensway Birmingham B1 1TT(GB)

## (54) Glycerol derivatives.

(57) A glycerol derivative of general formula:

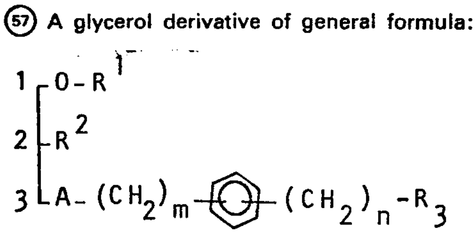

(1)

[wherein A represents an oxygen atom or a carbonyloxy group (wherein the carbonyl group should be attached to the group represented by $(CH_2)_m$ and the oxa group should be attached to the carbon atom at the 3rd position of the glycerol skelton), $R^1$ represents an alkyl group of 6 to 22 carbon atoms or an alkyl group of 2 to 5 carbon atoms being substituted by a phenyl group, $R^2$ represents a hydroxy group, an amino group, an azido group, an alkyl group of 1 to 20 carbon atom(s), an alkoxy group of 1 to 20 carbon atom(s) or a group of general formula:

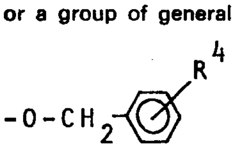

,

$-OCOR^5$, $-OCONHR^5$, $-NHCOR^5$, $-NHCOOR^5$ or $-NHCONHR^5$ (wherein $R^4$ represents a hydrogen atom, a halogen atom, an alkyl group of 1 to 4 carbon atom(s) or an alkoxy group of 1 to 4 carbon atom(s) and $R^5$ represents an alkyl group of 1 to 10 carbon atom(s) or a phenyl group), $R^3$ represents a group of general formula: $-N(R^6)_2$ or $-N^+(R^6)_3.Y^-$ (wherein each $R^6$ represents an alkyl group of 1 to 8 carbon atom(s) and which may be same or different to each other, or represents a heterocyclic ring of 4 to 10 ring members combining with 2 or 3 of $R^6$ and the nitrogen atom and if another $R^6$ not related to the heterocyclic ring exist, the $R^6$ represents an alkyl group of 1 to 8 carbon atom(s), and $Y^-$ represents an anion of acid.), m and n represent zero or an integer of from 1 to 6, respectively, and total number of m plus n should be more than 1. With the proviso that when A represents a carbonyloxy group, $R^2$ does not represent an amino group.] or an acid additional salt thereof have antagonistic effect on PAF, hypotensive effect like PAF inhibitory effect on phospholipase and further, inhibitory effect growth of tumour cell, and are therefore useful as a platelet aggregation inhibitor, anti-asthmatic agent, anti-allergic agent, anti-inflammatory agent, hypotensive agent and anti-tumour agent for mammals.

EP 0 145 303 A1

# DESCRIPTION

## GLYCEROL DERIVATIVES

### Summary

The present invention is concerned to new glycerol derivatives. More particularly, this invention is concerned to new glycerol derivatives having antagonistic effect on platelet activating factor (abbreviated as PAF hereafter), hypotensive effect like PAF, inhibitory effect on phspholipase and further, inhibitory effect on growth of tumour cell of induction of differentiation, and furthermore, this invention is concerned to the process for their preparation and pharmaceutical compositions containing them as active ingredients.

### Background

Recently, the study on the release reaction of active amine dependent on leukocyte from platelet, has been energetically carried out. As a result, a substance strongly inducing platelet aggregation was discovered and it was named as PAF [cf. J. Immunol., 106, 1244 (1971), J. Exp. Med., 136, 1356 (1972) and Nature, 249, 581 (1974)]. Thereafter, it was confirmed that PAF exists in various animals including human beings. In 1979, its chemical structure was identified [cf. J. Biol. Chem., 254, 9355 (1979) and C. R. Acad. Sci., Sefie D

(Paris), 289, 1017 (1979)] and has turned out to be a mixture of two alkylphospholipids of the following general formula wherein n is 15 or 17 [cf. J. Biol. Chem., 255, 5514 (1980)]:

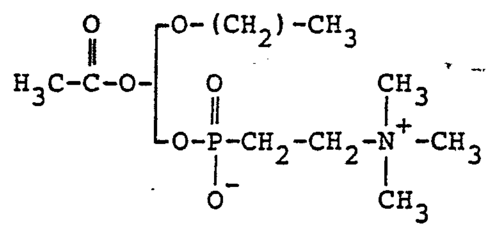

(wherein n represents 15 or 17.)

It was found that PAF has not only strong secretory effect on platelet aggregation known at that time, but also powerful hypotensive effect and bronchoconstricting effect as physiological effects. PAF is considered to be one of platelet aggregating factors in human beings, and further, one of substances inducing allergy and inflammation. Accordingly, there is a great possibility that compounds having antagonistic effect on PAF (inhibitory effect on PAF) may become an precedented platelet aggregation inhibitor, anti-asthmatic agent, anti-allergic agent or anti-inflammatory agent. Furthermore, PAF is considered to become a hypotensive agent without inhibitory effect on platelet aggregation by selecting hypotensive effect out of various effect.

Prior arts

Being interested in physiological effect on PAF, we have carried out widespread investigations in order to

discover compounds similar to PAF in their chemical structure and possessing antagonistic effect on PAF or hypotensive effect like PAF. We have found that the above purpose can be accomplished by glycerol derivatives which are replaced the hydroxy group at the first position of the glycerol skeleton by an alkoxy group and are replaced the hydroxy group at the third position thereof by an alkoxy group substituted by a primary, secondary, tertiary or quaternary amine, preferebly tertiary or quaternary amine.

For example, our European Patent Publication No. 94,586 discloses the compounds of the general formula:

$$\left[\begin{array}{l} A^{1}-R^{11} \\ B^{1}-R^{21} \\ D^{1}-R^{31}-R^{41} \end{array}\right.$$

(wherein $A^1$ is -O- or -S-, $B^1$ is -O- or -S-, $D^1$ is -O- or -OCO-, $R^{11}$ is alkyl $R^{21}$ is H, benzyl, acyl, N-substituted amido when $B^1$ is -O-, or $R^{21}$ is alkyl, benzyl or alkoxy (thiocarbonyl) when $B^1$ is -S-, $R^{31}$ is alkylene and $R^{41}$ is amino, mono- or dialkyl-substituted amino or trialkyl-substituted ammonium) and our European Patent Publication No. 109,255 disclose the compounds of the general formula:

$$
\begin{array}{l}
\ulcorner O-R^{12} \\
\vdash R^{22} \\
\llcorner A^{2}-R^{32}-R^{42}
\end{array}
$$

(wherein $A^2$ is -O- or -OCO-, $R^{12}$ is alkyl, $R^{22}$ is azido, amino, acylamino, alkoxycarbonylamino or ureido, $R^{32}$ is alkylene and $R^{42}$ is amino, mono- or dialkyl-substituted amino or trialkyl-substituted ammonium).

Now, we have found that by inducing a phenylene group into the side chain of the 3rd position of the glycerol skelton, and by limiting optionally substituted amino group attached to the end of the 3rd position thereof the tertiary or quaternary amine, new glycerol derivatives are most active in antagonistic effect on PAF than glycerol derivatives previously proposed, having completed this invention.

On the other hand, glycerol derivatives similar to those of the present invention in chemical structure are disclosed in the German Patent Publication No. 2,835,369. Broad scope of compound is disclosed therein and the compounds of the general formula:

$$
\begin{array}{l}
CH_2-O-R^{23} \\
| \\
CH-O-R^{13} \\
| \\
CH_2-(O-Y^1)_{mm}-(NH-Z^1)_{nn}-(CH_2)_{pp}NHR^{33}
\end{array}
\qquad (A)
$$

(wherein $R^{13}$ and $R^{23}$ each represents a normal alkyl or alkenyl group of 12 to 20 carbon atoms, $Y^1$ and $Z^1$ each

represent alkylene group of 2 to 4 carbon atoms, o-, m- or p-phenylenedimethylene group, a group of general formula:

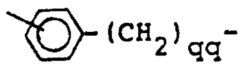 (wherein qq represents an integer of from 1 to 3 and the left bond was attached to the oxygen atom.) or a group of general formula:

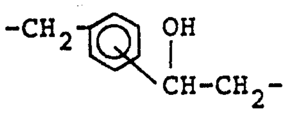 (wherein the left bond was attached to the oxygen atom.) or ...., $R^{33}$ represents a hydrogen atom, an alkyl group of 2 to 4 carbon atoms or ..., mm, nn and pp each represents zero or 1 and the total number of mm, nn and pp is zero or 1.) are most related to our invention. (The difinition not related to our invention is removed from the above description.)

As understood from the above definition, $-NHR_3$ in formula (A) in DE-2,835,369 represents only a primary or secondary amine, which $R^4$ in the general formula (I) hereinafter described, of the present invention represents a tertiary or quaternary amine.

Furthermore, the distinguished difference between the present invention and the invention disclosed in DE-2,835,369 is utility of glycerol compounds. That is, compounds of the present invention have antagonistic effect on PAF and hypotensive effect like PAF and, therefore, are used as platelet aggregation inhibitor, anti-asthmatic agent, anti-allergic agent and anti-inflammatory agent, whereas compounds in DE-2,835,369 have stimulating activity of production of interferon and are useful for prophylaxis of

viral disease.

As discussed above, the present invention is remarkably different from the invention disclosed in DE-2,835,369 in chemical structure and their utility. The present invention is not anticipated and not obvious from DE-2,835,369.

Additional activities

Furthermore, it has been found that the compound of the present invention of the general formula (I) have inhibitory effect on phospholipase $A_2$ and phospholipase C. Phospholipase $A_2$ and Phoapholipase C dependent on calcium in a living body are concerned in the mechanism of the release of arachidonic acid from phosphlipids. It is known that arachidonic acid is released by physiological stimulus and then become incorporated into the metabolic routes of prostaglandins and is finally metabolized to thromboxane $A_2$ having a strong effect on platelet aggegation, to other prostaglandins or to various leukotrienes being a significant bronchoconstrictor in an asthmatic paroxysm. Accordingly, it is considered that compounds having an inhibitory effect on phospholipase $A_2$ and phospholipase C, which are concerned in the mechanism for the release of arachidonic acid, can become a platelet aggregation inhibitor, anti-asthamatic agent, anti-allergic agent or anti-inflammatory agent, based on the inhibition of phospholipase $A_2$ and phospholipase C, having a different mechanism from PAF antagonist and further, being

different from conventional non-steroid anti-inflammatory agents represented by aspirin based on the inhibition of cyclooxygenase.

Furthermore, the compounds of the present invention of the general formula (I) show inhibitory effect on growth of tumour cell and induction of differentiation and ,therefore, are may be useful as anti-tumour agents.

Chemical structure

The present invention is concerned to new glycerol derivatives of the general formula:

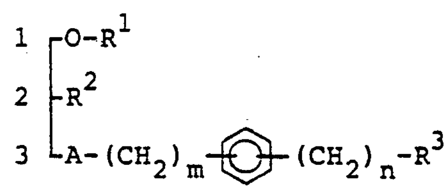

(I)

[wherein A represents an oxygen atom or a carbonyloxy group (wherein the carbonyl group i.e. -O-CO- group, should be attached to the group represented by $(CH_2)_m$ and the oxa group should be attached to the carbon atom at the 3rd position of the glycerol skelton), $R^1$ represents an alkyl group of 6 to 22 carbon atoms or an alkyl group of 2 to 5 carbon atoms being substituted by a phenyl group, $R^2$ represents a hydroxy group, an amino group ($-NH_2$ group), an azido group ($-N_3$ group), an alkyl group of 1 to 20 carbon atom(s), an alkoxy group of 1 to 20 carbon atom(s) or a group

of general formula: $-O-CH_2-$ 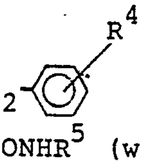 , $-OCOR^5$, $-OCONHR^5$, $-NHCOR^5$, $-NHCOOR^5$ or $-NHCONHR^5$ (wherein $R^4$ represents a hydrogen atom, a halogen atom, an alkyl group of 1 to 4 carbon atom(s) or an alkoxy group of 1 to 4 carbon atom(s) and $R^5$ represents an alkyl group of 1 to 10 carbon atom(s) or a phenyl group), $R^3$ represents a group of general formula: $-N(R^6)_2$ or $-N^+(R^6)_3.Y^-$ (wherein each $R^6$ represents an alkyl group of 1 to 8 carbon atom(s) and which may be same or different to each other, or represents a heterocyclic ring of 4 to 10 ring members combining with 2 or 3 of $R^6$ and the nitrogen atom and if another $R^6$ not related to the heterocyclic ring exist, the $R^6$ represents an alkyl group of 1 to 8 carbon atom(s), and $Y^-$ represents an anion of acid.), m and n represent zero or an integer of from 1 to 6, respectively, and total number of m plus n should be more than 1. With the proviso that when A represents a carbonyloxy group, $R^2$ does not represent an amino group.] and acid additional salts thereof.

Throughout the specification including claims, the term "alkyl" or "alkyl" in term of alkoxy group means "a straight- or branched-chain alkyl", respectively.

Examples of the alkyl group of 6 to 22 carbon atoms representsed by $R^1$ are hexyl, heptyl, octyl, nonyl, decyl, undecyl, dodecyl, tridecyl, teradecyl, pentadecyl, hexadecyl, heptadecyl, octadecyl, nonadecyl, eicocyl, heneicocyl, dococyl group and isomers thereof. Preferably, an alkyl group of 14

to 18 carbon atoms and hexadecyl group is most preferred.

Examples of the alkyl group of 2 to 5 carbon atoms substituted by a phenyl group, represented by $R^1$ are ethyl, propyl, butyl, pentyl groups and isomers thereof.

Examples of the alkyl group of 1 to 20 carbon atom(s) represented by $R^2$, in the general formula (I), are methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonedecyl, eicocyl groups and isomers thereof. And an alkyl group of 1 to 6 carbon atom(s) is preferred.

Examples of the alkoxy group of 1 to 20 carbon atom(s) represented by $R^2$, are alkoxy groups corresponding to the alkyl groups mentioned above and an alkoxy group of 1 to 6 carbon atom(s) is preferred.

Examples of the substituents represented by $R^4$, in the general formula:

$-O-CH_2-$ 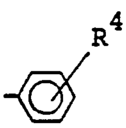 , as $R^2$ are a hydrogen atom, a fluorine atom, a chlorine atom, a bromine atom ,an iodine atom and methyl, ethyl, propyl, butyl, methoxy, ethoxy, propoxy, butoxy groups and isomers thereof. Preferably, a hydrogen atom, a chlorine atom, a methyl group and a methoxy group and a hydrogen atom is most preferred.

Examples of the alkyl group of 1 to 10 carbon atom(s) represented by $R^5$ in the general formula: $-OCOR^5$, $-OCONHR^5$, $-NHCOR^5$, $-NHCOOR^5$ or $-NHCONHR^5$ as $R^2$ are methyl, ethyl, propyl, butyl, pentyl, hexyl, heptyl,

octyl, nonyl, decyl groups and isomers thereof. Preferably, an alkyl group of 1 to 6 carbon atom(s) i.e. methyl, ethyl , propyl, isopropyl, butyl, isobutyl, sec-butyl, tert-butyl group, and pentyl and hexyl groups and isomers thereof. A phenyl group as $R^5$ is also preferred.

Any group as $R^2$ is also preferred and a hydroxy group, an amino group, an azido group and a group of general formula: $-OCOR^5$, $-NHCOR^5$, $-NHCOOR^5$ or $-NHCONHR^5$ (wherein all the symbols are as defined hereinbefore.) are most preferred.

Examples of the alkyl group of 1 to 8 carbon atom(s) represented by $R^6$ as $R^3$ in the general formula (I), are methyl, ethyl, propyl, butyl, pentyl, hexyl, heptyl, octyl groups and isomers thereof and an alkyl group of 1 to 4 carbon atom(s) is preferred.

The heterocyclic ring combining 2 or 3 of $R^6$ and the nitrogen atom is meant that:

(1) ring members is 4 to 10.

(2) it contains one nitrogen atom or, one nitrogen and another one or two nitrogen, oxygen or sulphure atoms (with the proviso that total number of the hetero atoms should be less than 3.); and contains carbon atoms not less than 2.

(3) single ring or bridged double ring.

(4) it has double bond(s) or not in the ring, or is an aromatic or non-aromatic heterocyclic one.

(5) it may be substituted by 1 to 5 of alkyl group(s) of 1 to 8 carbon atom(s) (e.g. methyl, ethyl, propyl, butyl, pentyl,

hexyl group or isomers thereof; preferably a methyl group.) at any position of the ring; preferably, at the nitrogen atom.

Examples of such the heterocyclic ring are 1-azetidinyl, 1-pyrrolidinyl, hexamethyleneimino, heptamethyleneimino, 1-imidazolidinyl, 1-piperazinyl, 1-pyrrolyl, 1-imidazolyl, 1-pyridinio, 1-pyrazinio, 1-quinuclidinio, triethylenediamin-1-io, 3-oxazolio, morpholino, thiazolio groups and N-alkyl substituted ones thereof.

The anions of acid represented by $Y^-$ mean the anions of pharmaceutically-acceptable non-toxic inorganic or organic acid, and examples of them are the anions of inorganic acid such as hydrochloric acid, hydrobromic acid, hydroiodic acid, sulphuric acid, phosphoric acid, nitric acid and the anions of organic acid such as acetic acid, lactic acid, tartaric acid, benzoic acid, citric acid, methanesulphonic acid, ethanesulphonic acid, benzenesulphonic acid, toluenesulphonic acid, isethionic acid.

Preferred anion is a halogen ion, i.e. chlorine, bromine and iodine ions or an anion of methanesulphonic acid or p-toluenesulphonic acid.

Zero or any integer of from 1 to 6 as m or n is also preferred.

A phenylene group represented by a formula: 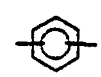 represents o-, m- or p-phenylene group, and a p-phenylene group is preferred.

The compound of the general formula (I) have at

least one asymmetric center, i.e. the carbon atom at the second position of the glycerol. When the alkyl moiety of various substituents represented by $R^1$, $R^2$ or $R^3$ represents a branched-chain, there is a possibility that other asymmetric centers are occurred. However, the general formula (I) in the present invention include each isomers occurred by asymmetric carbon(s) and mixtures thereof.

<u>Processes for the preparation</u>

According to a feature of the present invention, compounds of the general formula (I) wherein A represents an oxygen atom and $R^2$ represents other than an amino group or a group of general formula: $-OCOR^5$ or $-OCONHR^5$ (wherein $R^5$ is as defined hereinbefore.) i.e. compounds of general formula:

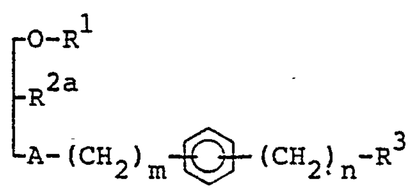

(Ia)

/wherein $R^{2a}$ represents a hydroxy group, an azido group, an alkyl group of 1 to 20 carbon atom(s), an alkoxy group of 1 to 20 carbon atom(s) or a group of general formula:

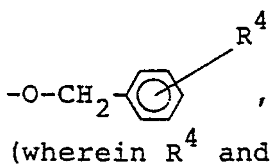
, $-NHCOR^5$, $-NHCOOR^5$ or $-NHCONHR^5$ (wherein $R^4$ and $R^5$ are as defined hereinbefore.) and the other symbols are as defined hereinbefore./

may be prepared by reaction of a compound of general formula:

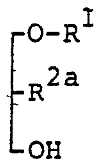

(II)

[wherein all the symbols are as hereinbefore defined.]
with a compound of general formula:

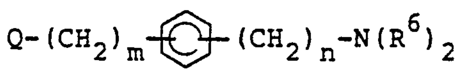

(III)

[wherein Q represents a halogen atom or an alkylsulphonyloxy group or an arylsulphonyloxy group being substituted or unsubstituted (preferably a mesyloxy group or a tosyloxy group .) and the other symbols are as defined hereinbefore.]
and further, if desired, subjecting the obtained compound of general formula:

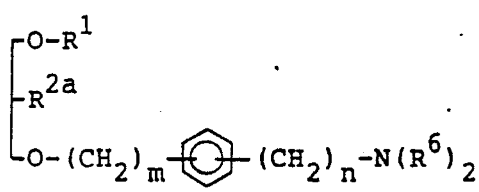

(Ia-1)

[wherein all the symbols are as defined hereinbefore.]
to alkylation, and further, if desired, subjecting the obtained compound of general formula:

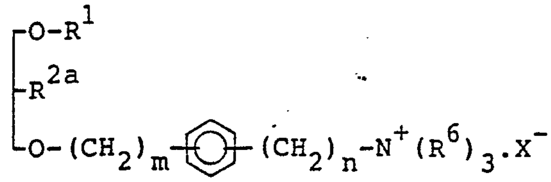

(Ia-2)

[wherein X represents a halogen atom and the other symbols are as defined hereinbefore.]

to a method for salt-exchange.

The reaction of the compound of the general formula (II) and the compound of the general formula (III) may be carried out in the presence of a suitable base e.g. a hydride of alkaline metal such as butyl lithium, potassium tert-butoxide, in an inert organic solvent such as demethylformamide (DMF), benzene, toluene or tetrahydrofuran (THF), at from room temperature to 100°C, for from 30 minutes to several days.

Equivalent molar or more of the compound of the general formula (III) is used preferably to the amount of the compound of the general formula (II) used.

Alkylation of the compound of the general formula (Ia-1) may be carried out in a lower alkanol such as methanol, ethanol, isopropanol, with using an alkyl halide such as methyl iodide, from room temperature to a refluxing temperature of solvent used.

The compound of the general formula (Ia-2) may be converted to a salt which contained an anion of acid other than a halogen atom by a method for salt-exchange.

Salt-exchange may be carried out by known method, that is (i)

by reacting the compound of the general formula (Ia-2) with a desirable inorganic acid or organic acid after neutrilizing, by an aqueous solution of sodium hydroxide, or (ii) with using an ion exchange resin.

Compounds of the general formula (I) wherein A represents an oxygen atom and $R^2$ represents an animo group i.e. compounds of general formula:

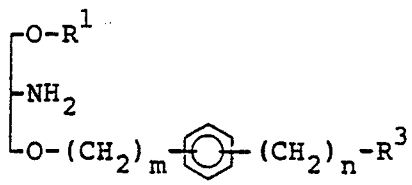

(Ib)

/wherein all the symbols are as defined hereinbefore./
may be prepared by reaction of a compound of general formula:

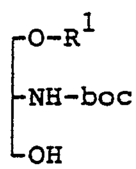

(IV)

/wherein boc represents a tert-butoxycarbonyl group and the other symbols are as defined hereinbefore./
with the compound of the general formula (III), and further, if desired, subjecting the obtained compound to alkylation to obtain compound of general formula:

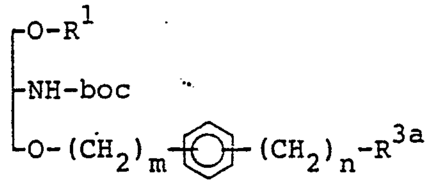

(V)

/wherein $R^{3a}$ represents a group of general formula: $-N(R^6)_2$ or $-N^+(R^6)_3.X^-$ (wherein all the symbols are as defined hereinbefore.) and the other symbols are as defined hereinbefore./
and further, removal of boc group of the obtained compound to obtain compound of general formula:

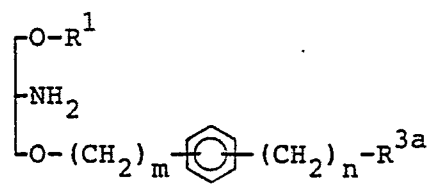

(Ib-1)

/wherein all the symbols are as defined hereinbefore./
and further, if desired, subjecting the obtained compound to a method for salt-exchange.

The reaction of the compounds of the general formula (IV) and the compounds of the general formula (III) may be carried out by the same method as the reaction of the compounds of the general formula (II) and the compounds of the general formula (III) above mentioned, and the alkylation may also be carried out by the same method above mentioned.

Removal of boc group is a known reaction, in an inert organic solvent such as ethyl acetate, methylene chloride, chloroform or a lower alkanol, with using

hydrochloric acid at from room temperature to 50°C or with using trifluoroacetic acid at room temperature.

Salt-exchange may be carried out by the same method above mentioned.

Compounds of general formula (I) wherein A represents an oxygen atom and $R^2$ represents a group of general formula: $-OCOR^5$ or $-OCONHR^5$ (wherein $R^5$ is as defined hereinbefore.), i.e. compounds of general formula:

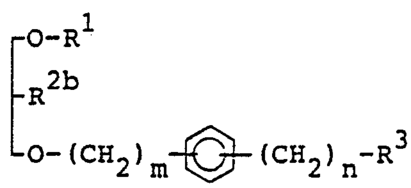

(Ic)

[wherein $R^{2b}$ represents a group of the general formula: $-OCOR^5$ or $-OCONHR^5$ (wherein $R^5$ is as defined hereinbefore.) and the other symbols are as defined hereinbefore.]

may be prepared by reaction of a compound of general formula:

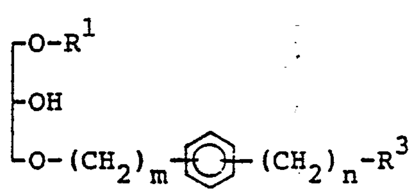

(Ia-3)

[wherein all the symbols are as defined hereinbefore.]

with an acid of general formula:

$$HOOC-R^5 \quad (VI)$$

[wherein $R^5$ is as defined hereinbefore.]
or an active derivative thereof (i.e. esterification)
or an isocyanate of general formula:

$$O=C=N-R^5 \quad (VII)$$

[wherein $R^5$ is as defined hereinbefore.]

The esterification of the compound of the general formula (Ia-3) and the acid of the general formula (VI) or active derivative thereof may be carried out by known method. That is, in an inert organic solvent such as ethyl acetate, benzene, diethyl ether, chloroform, methylene chloride, tetrahydrofuran or a mixture of two or more of them, at from -10°C to 50°C (preferably, at from 0°C to room temperature).

Examples of the active derivatives of acids of the general formula (VI) are an acid halide such as acid chloride, acid bromide, acid anhydride, or a mixed acid anhydride with pivaloyl chloride, isobutyl chloride or isobutyl chloroformate, an active ester with dicyclohexylcarbodiimide (DCC). The reaction is preferably carried out in the presence of a base such as pyridine, 4-dimethylaminopyridine or triethylamine.

Equivalent molar or more of the compound of the general formula (VI) is used preferably to the amount of the compound of the general formula (Ia-3) used.

The reaction of the compound of the general formula (Ia-3) and the compound of the general formula (VII) may be carried out in an inert organic solvent such as ethyl acetate, benzene, hexane, diethyl ether, tetrahydrofuran, chloroform, methylene chloride, carbon tetrachloride or a mixture of two or more of them, in the presence of a tertiary amine such as pyridine, triethylamine, at from 0°C to 50°C, with using an isocyanate of the general formula (VII).

Further according to the present invention, compounds of the general formula (I) wherein A represents a carbonyloxy group and $R^2$ represents other than an amino group , i.e. compounds of general formula:

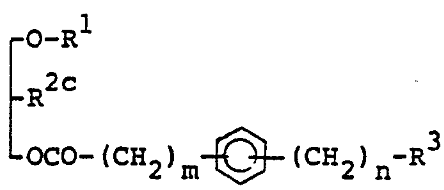

(Id)

[wherein $R^{2c}$ represents a hydroxy group, an azido group, an alkyl group of 1 to 20 carbon atom(s), an alkoxy group of 1 to 20 carbon atom(s) or a group of general formula:

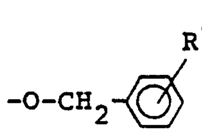 , $-OCOR^5$, $-OCONHR^5$, $-NHCOR^5$, $-NHCOOR^5$ or $-NHCONHR^5$ (wherein $R^4$ and $R^5$ are as defined hereinbefore.) and the other symbols are as defined hereinbefore.]

may be prepared by esterification of a compound of general

formula:

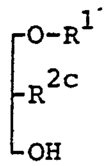

(VIII)

/wherein all the symbols are as defined hereinbefore./
with an acid of general formula:

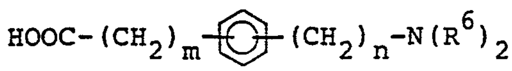

(IX)

/wherein all the symbols are as defined hereinbefore./
or an active derivative thereof, and further, if desired, subjecting to alkylation and further, subjecting to a method for salt-exchange if desired.

Esterification, alkylation and salt-exchange may be carried out by the same method mentioned above, respectively.

Further according to the present invention, compounds of the general formula (I) wherein A represents an oxygen atom, $R^2$ represents a group of general formula:

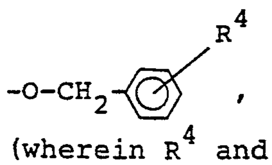

, $-NHCOR^5$, $-NHCOOR^5$ or $-NHCONHR^5$
(wherein $R^4$ and $R^5$ are as defined hereinbefore.) and n represents an integer of from 1 to 6 and the other symbols are defined hereinbefore, i.e. compounds of general formula:

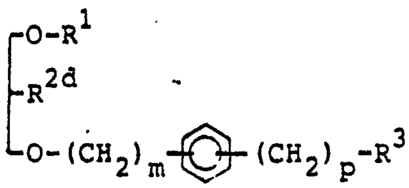

(Ie)

[wherein $R^{2d}$ represents a group of general formula:

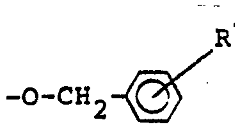 , $-NHCOR^5$, $-NHCOOR^5$ or $-NHCONHR^5$

(wherein $R^4$ and $R^5$ are as defined hereinbefore.) and p represents an integer of from 1 to 6 and the other symbols are as defined hereinbefore.]

may be prepared by reaction of a compound of general formula:

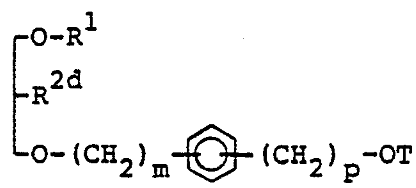

(X)

[wherein T represents an alkylsulphonyl group or an arylsulphonyl group being substituted or unsubstituted (preferably a mesyl group or a tosyl group) and the other symbols are as defined hereinbefore.]

with a secondary amine of general formula:

$$NH(R^6)_2 \quad (XI)$$

[wherein $R^6$ is as defined hereinbefore.]

and further, if desired, converting the obtained tertiary amine into a quaternary one by alkylation , and further, if

desired, subjecting the obtained quaternary ammonium salt to a method for salt-exchange; or, may be prepared by reaction of a compound of the general formula (X) with a tertiary amine of general formula:

$$N(R^6)_3 \qquad (XII)$$

[wherein $R^6$ is as defined hereinbefore.]
and further, if desired, subjecting the obtained quaternary ammonium salts to a method for salt-exchange.

The reaction of the compound of the general formula (X) and the amine of the general formula (XI) or (XII) may be carried out in a suitable inert organic solvent, e.g. a lower alkanol such as methanol, ethanol, isopropanol, DMF or a mixture of two or more of them, at from room temperature to a refluxing temperature of solvent used, for from one hour to several days.

Equivalent molar or more of the amine is used preferably to the amount of the compound of the general formula (X) used.

Esterification, alkylation and salt-exchange may be carried out by the same method mentioned above, respectively.

Starting materials

The compounds of the general formula (III), (VI) (VII), (IX), (XI) and (XII) are known compounds *per se* or may be prepared easily by known method.

The compounds employed as starting materials of the general formula (II) and (VIII) wherein $R^{2a}$ or $R^{2c}$ represents a hydroxy group, an azido group or a group of general formula:

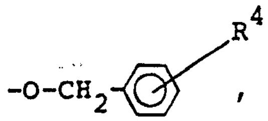 , $-NHCOR^5$, $-NHCOOR^5$ or $-NHCONHR^5$

(wherein $R^4$ and $R^5$ are as defined hereinbefore.) or $R^{2c}$ represents a group of general formula: $-OCOR^5$ (wherein $R^5$ is as defined hereinbefore.)

may be prepared by the method described in the specification of European Patent Publication No. 94,586 (abbreviated as Literature "A" hereafter) which proposed previously by the present inventors, or by obvious similar methods thereof.

And the compounds of the general formula (II) and (VIII) wherein $R^{2a}$ or $R^{2c}$ represents an alkyl group of 1 to 20 carbon atom(s) or an alkoxy group of 1 to 20 carbon atom(s) may be prepared by the method described in the specification of Japanese Patent Application No. 58-208,355 filed on 8th December, 1983 or by obvious similar methods thereof.

The compounds employed as a starting material of the general formula (VIII) wherein $R^{2c}$ represents a group of general formula: $-OCONHR^5$ (wherein $R^5$ is as defined hereinbefore.) may be prepared by reaction of a compound of general formula:

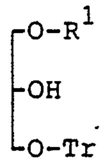

(XIII)

[wherein Tr represents a trityl group and $R^1$ is as defined hereinbefore.]

with the compound of the general formula (VII), by the method described hereinbefore and further removal of a trityl group of the obtained compound by treating in an aqueous solution of acid.

And the compounds employed as a starting material of the general formula (IV) may be prepared by protection of an animo group by a boc group, of a compound of general formula:

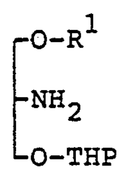

(XIV)

[wherein THP represents a tetrahydropyran-2-yl group and $R^1$ is as defined hereinbefore.]

with tert-butyl dicarbonate in an aqueous solution of sodium hydroxide at room temperature or in methylene chloride in the presence of triethlyamine at room temperature or below, and further removal of the THP group of the obtained compound by the method described hereafter.

The reaction should be carried out carefully in order to avoid elimination of the boc group, at the same time.

Each of the compound of the general formula (XIII) and (XIV) above mentioned are the ones described in the literature A.

And the compounds employed as a starting material of the general formula (X) [i.e. compounds of general formula from (XA-1) to (XA-3) and from (XB-1) to (XB-4) described hereafter.]
may be prepared by series of the reactions in the following scheme A and B.

In the Schemes, q represents an integer of from 1 to 6, r represents zero or an integer of from 1 to 4, s represents an integer of from 2 to 6,t represents zero or an integer of from 1 to 3, u represents an integer of from 3 to 6, $R^7$ represents a lower alkyl group (preferably an ethyl group), $R^{2e}$ represents a group of general formula:
$-NHCOR^5$, $-NHCOOR^5$ or $-NHCONHR^5$ (wherein $R^5$ is as defined hereinbefore.) and the other symbols are as defined hereinbefore.

Scheme A

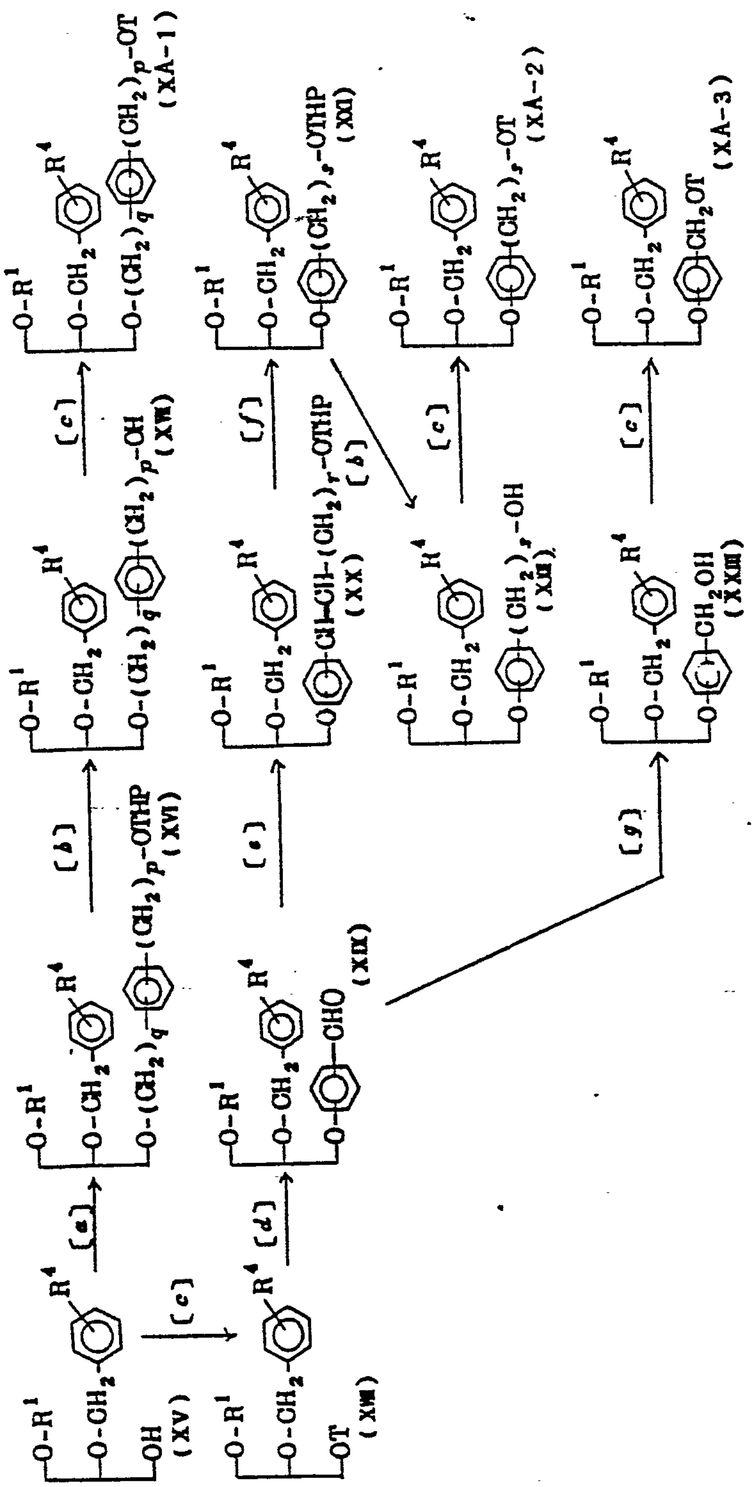


(XV) (XVII) (XVI) (XIX) (XVIII) (XX) (XXII) (XXIII) (XA-1) (XXI) (XA-2) (XA-3)

Scheme B

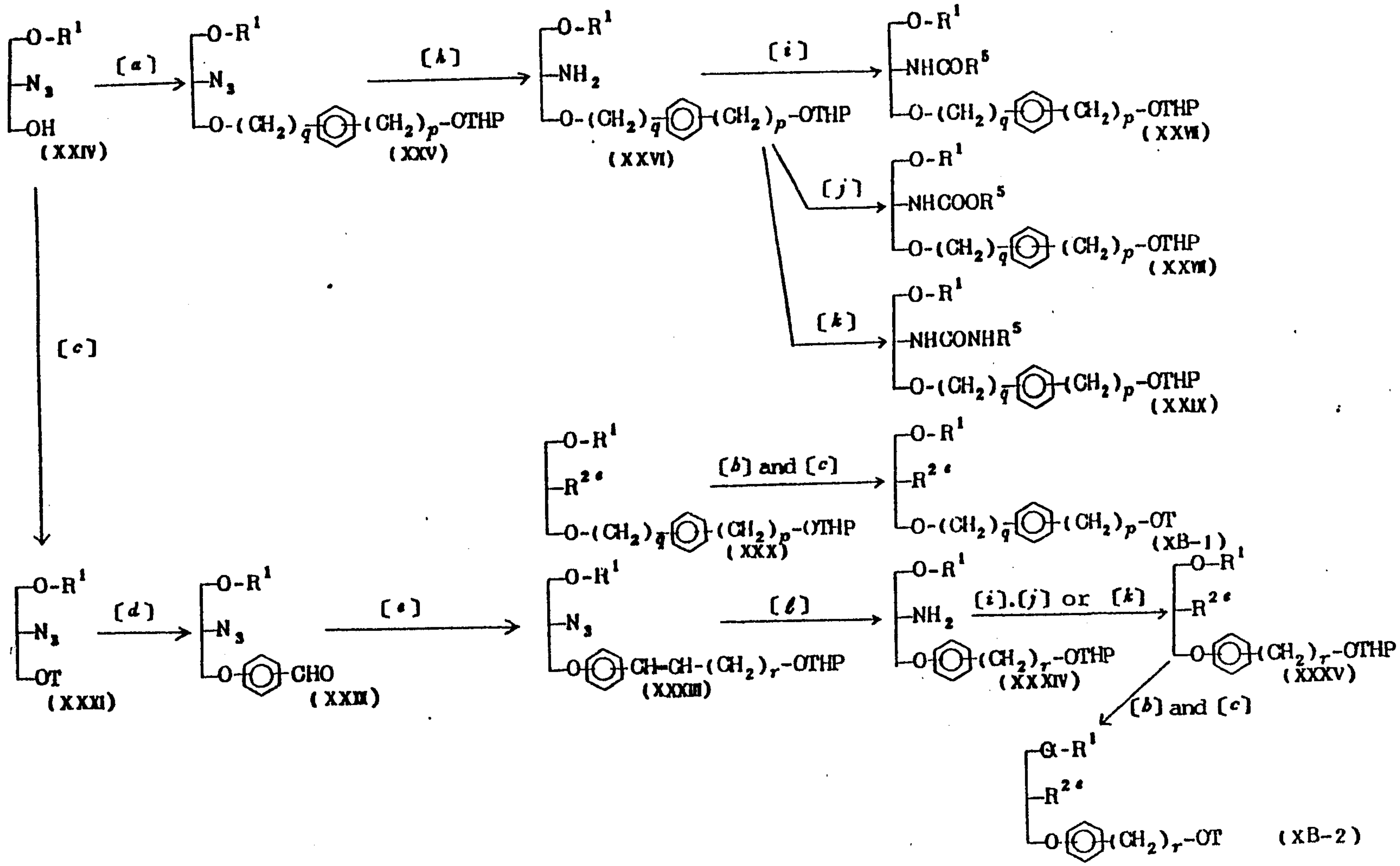
(XXIV)
[a]
(XXV)
[h]
(XXVI)
[i]
(XXVII)
[j]
(XXVIII)
[k]
(XXIX)
[c]
(XXXI)
[d]
(XXXII)
[e]
(XXXIII)
[ℓ]
(XXXIV)
[i],[j] or [k]
(XXXV)
[b] and [c]
(XXX)
[b] and [c]
(XB-1)
[b] and [c]
(XB-2)

Scheme B (continuation)

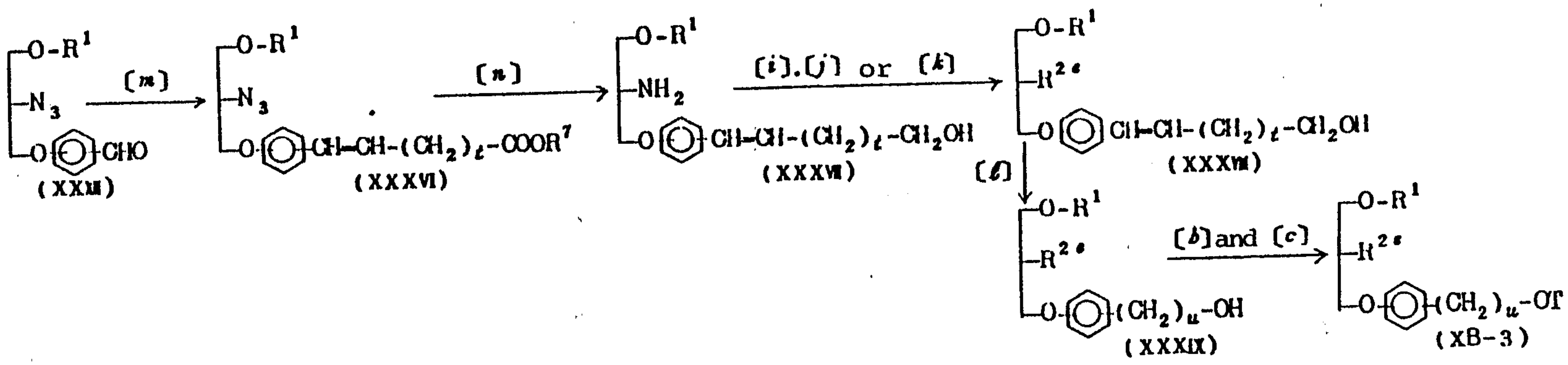

(XXXII) (XXXVI) (XXXVII) (XXXVIII) (XXXIX) (XB-3)

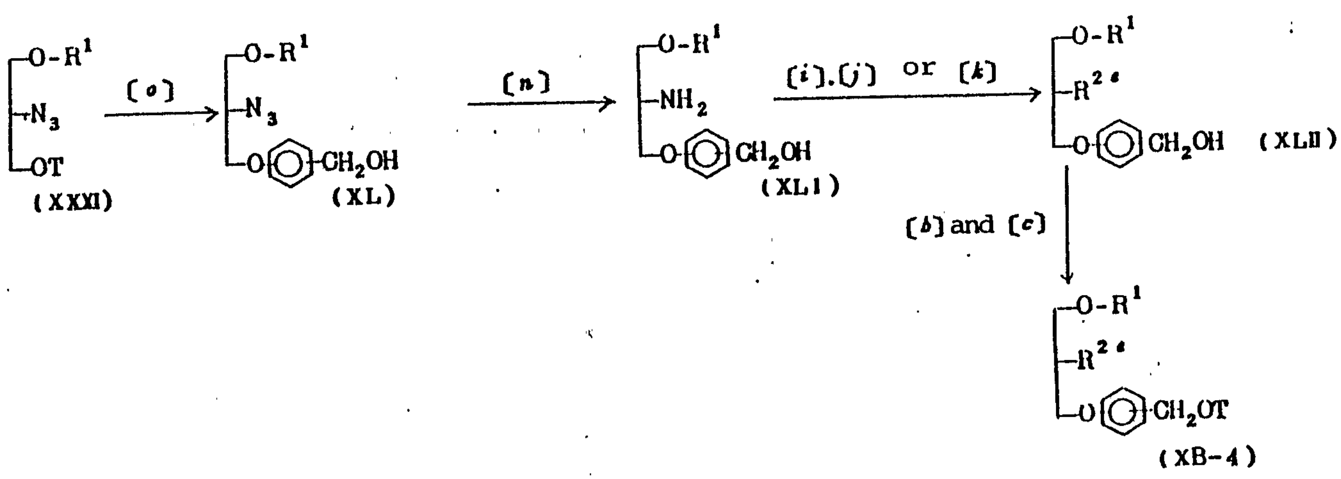

(XXXI) (XL) (XLI) (XLII) (XB-4)

Each steps in the schemes may be carried out by known method. For example, step [a] may be carried out by the same method as the reaction of the compound of the general formula (II) and the compound of the general formula (III) described hereinbefore using with the compound of the general formula (XV) and a compound of general formula:

Q-$(CH_2)_q$ 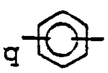 $(CH_2)_p$-OTHP (XLIII)

[wherein all the symbols are as defined hereinbefore.]

Step [b] is detetrahydropyranation, and it may be carried out using a mixture of tetrahydrofuran, acetic acid and water, a mixture of p-toluenesulphonic acid and anhydrous methanol or a mixture of diluted hydrochloric acid and tetahydrofuran, at room temperature or with heating.

Step [c] may be carried out, reacting with alkylsulphonyl chloride such as mesyl chloride or arylsulphonyl chloride such as tosyl chloride, at from -30°C to 50°C, (i) in the presence of a tertiary amine such as pyridine, triethylamine in an inert organic solvent such as methylene chloride, or (ii) in pyridine. And equivalent molar or more of alkyl- sulphonyl chloride or arylsulphonyl chloride used was preferred.

Step [d] may be carried out, reacting with a compound of general formula (XVIII) and a compound of general formula:

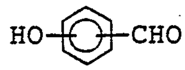 (XLIV)

e.g. p-hydroxybenzaldehyde, in the presence of weak base such as sodium carbonate, potassium carbonate, in an inert organic solvent such as DMF, benzene, toluene, THF, acetone, at from room temperature to 100°C.

Step [e] is Wittig reaction, it may be carried out in an inert organic solvent such as dimethyl sulphoxide, THF, using with a phosphonium ylide of general formula:

$$Ø_3P^+-(CH_2)_{r+1}-OTHP.Br^- \qquad (XLV)$$

[wherein Ø represents a phenyl group.]

The ylide may be prepared by reacting the compound of the general formula (XLV) in the presence of a suitable base such as butyl lithium.

Step [f] is selective reduction of the vinylene group in the side-chain of the 3rd position, into an ethylene group. For example, the method is described in Chapter 1 of "Modern Synthetic Reactions" 2nd edition, 1972, written by H.O. House, published by W.A. Benjamin Inc.) in detail.

Step [g] is reduction of the formyl group into a hydroxymethyl group and it may be carried out in a suitable inert organic solvent such as THF, diethyl ether, lower alkanol, using with a reducing agent such as lithium aluminium hydride, sodium borohydride at from 0°C to a refluxing temperature of solvent used.

Step [h] is reduction and it may be carried out: (i)

in a suitable inert organic solvent e.g. THF, diethyl ether, using with lithium aluminium hydride at from 0°C to a refluxing temperature of solvent used or (ii) when q and p represent an integer other than 1, in an atmosphere of hydrogen in the presence of catalyst such as palladium-carbon, or palladium black, in an inert organic solvent e.g. a lower alkanol such as methanol, ethanol, ethyl acetate or a mixture of two or more of them, preferably in the presence of an acid such as acetic acid, hydrochloric acid, hydroiodic acid, methanesulphonic acid, at from room temperature to a refluxing temperature of solvent used.

Step /i/ may be carried out in an inert organic solvent e.g. ethyl acetate, benzene, hexane, diethyl ether, tetrahydrofuran, chloroform, methylene chloride, carbon tetrachloride or a mixture of two or more of them, or without solvent, in the presence of a tertiary amine such as pyridine, triethylamine, by reacting with the amine of the general formula (XXVI) and an acyl halide of general formula:

$$X^1\text{-COR}^5 \qquad \text{(XLVI)}$$

/wherein $X^1$ represents a halogen atom, and $R^5$ is as defined hereinbefore./
or an acid anhydride of general formula:

$$R^5\text{-COO-CO-}R^5 \qquad \text{(XLVII)}$$

[wherein $R^5$ is as defined hereinbefore.]
at from 0°C to 50°C.

Step [j] may be carried out by the same procedure as step [i], using a haloformic acid ester of general formula:

$$X^2\text{-COOR}^5 \qquad \text{(XLVIII)}$$

[wherein $X^2$ represents a halogen atom and $R^5$ is as defined hereinbefore.]
instead of the compound of the general formula (XLVI) or (XLVII).

Step [k] may be carried out by the same procedure as step [i], using the isocyanate of the general formula (VII), in stead of the compound of the general formula (XLVI) or (XLVII).

Step [l] may be carried out by the same procedure as (2) of step [h].

Step [m] is Wittig reaction, and it may be carried out by the same procedure as step [e], using a sodio derivative [it may be prepared by a phosphonate of the general formula (XLIX) and sodium hydride.] of phosphonate of general formula:

$$(C_2H_5O)_2\underset{\underset{O}{\|}}{P}-(CH_2)_{t+1}-COOR^7 \qquad (XLIX)$$

/wherein t and $R^7$ are as defined hereinbefore./
in stead of the phosphonium ylide of the general formula (XLV).

Step /n/ may be carried out by the same procedure as (1) of step /h/.

Step /o/ may be carried out by the same procedure as step /d/, using a compound of general formula:

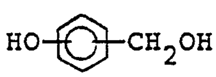

(L)

in stead of the compound of the general formula (XLIV).

The compounds of the general formula (XV) and (XXIV) employed as starting materials in the schemes above, are described in the Literature A. And the compounds of the general formula from (XLVIII) to (L) are also known compounds per se or may be prepared easily by known methods per se.

And further according to the present invention, compounds of general formula:

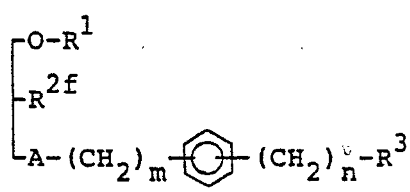

(If)

/wherein (i) when A represents an oxygen atom, $R^{2f}$

represents an amino group, an azido group or a group of general formula:
$-NHCOR^5$, $-NHCOOR^5$ or $-NHCONHR^5$ (wherein $R^5$ is as defined hereinbefore.) and the other symbols are as defined hereinbefore.]
among the glycerol derivatives of the general formula (I), may also be prepared by the series of reactions depicted in the following scheme C, wherein the various symbols are as defined hereinbefore.

Scheme C

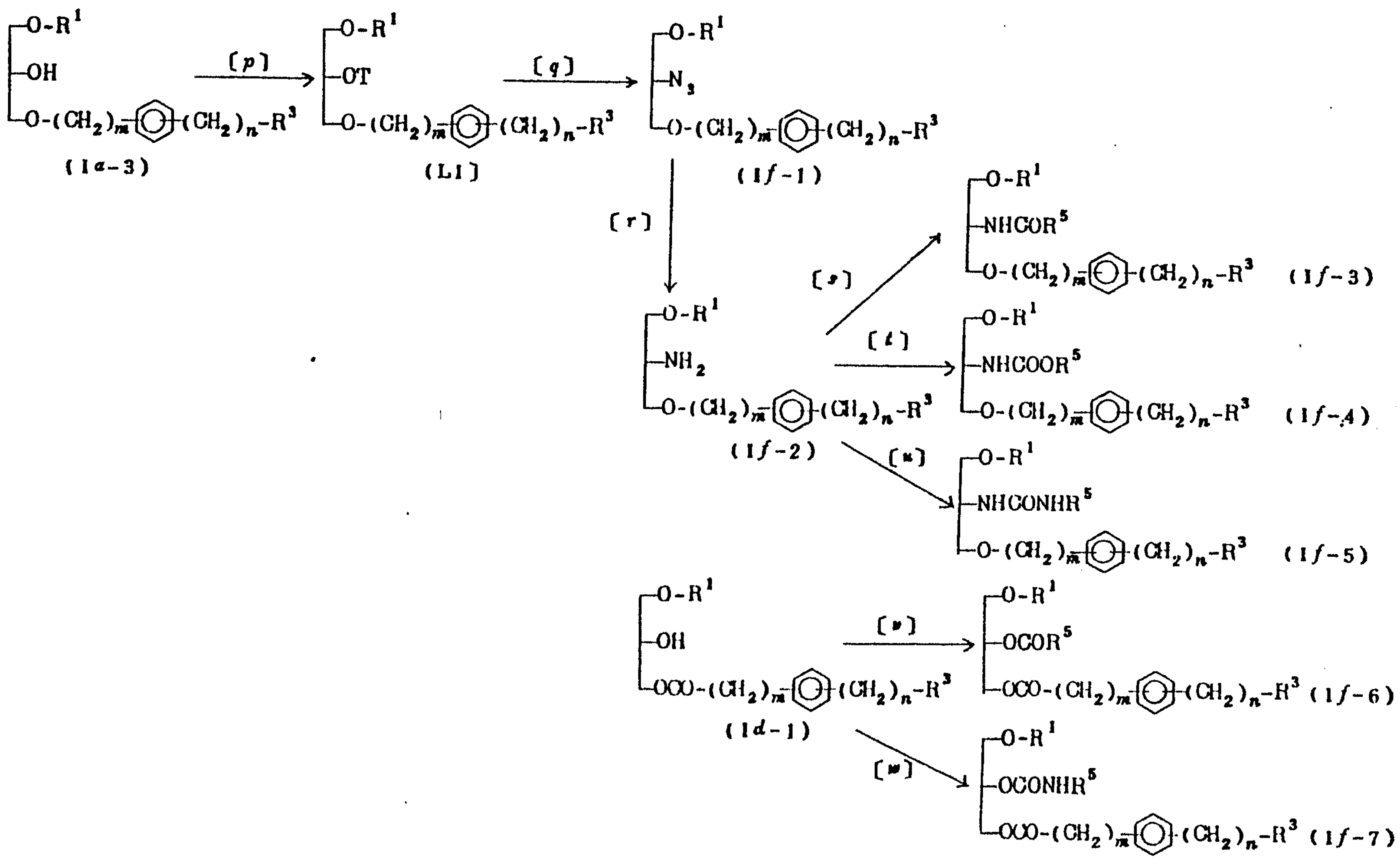


(1a-3) (L1) (1f-1) (1f-2) (1f-3) (1f-4) (1f-5) (1d-1) (1f-6) (1f-7)

Each steps in the scheme C may be carried out by the same procedure as described hereinbefore or by the methods known *per se*. For example, step /p/ may be carried out by the same procedure as step /c/.

Step /q/ may be carried out in an inert organic solvent such as hexamethylphospharamide, DMF, dimethylsulphoxide, at from room temperature to 80°C, using with sodium azide.

Step /r/, /s/, /t/ and /u/ may be carried out by the same procedure as steps /h/, /i/, /j/ and /k/, respectively.

Step /v/ and /w/ may be carried out by the same procedures as the reaction of the compound of the general formula (Ia-3) and the compound of the general formula (VI), and the reaction of the compound of the general formula (Ia-3) and the compound of the general formula (VII) described previously, respectively.

And further according to the present invention, compounds of general formula:

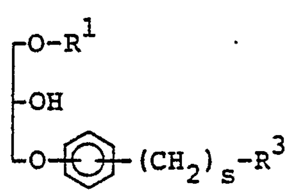

(Ig)

/wherein all the symbols are as defined hereinbefore./ among the glycerol derivatives of the general formula (I) may also be prepared by removal of a benzyl group attached to the

2nd position of a compound of general formula (LII) and conversion into an ethylene group, of a vinylene group in a side chain of the 3rd position of general formula:

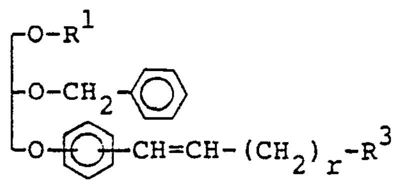

(LII)

[wherein all the symbols are as defined hereinbefore.] at the same time, by the same procedure as (2) of step [h].

The compounds of the general formula (LII) may be prepared by the same procedures as step [b] and followed by step [c], using the compound of the general formula (XX) wherein $R^4$ represents a hydrogen atom to obtain compound of general formula:

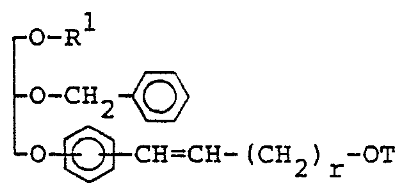

(LIII)

[wherein all the symbols are as defined hereinbefore.] and further reacting the obtained compound and the compound of the general formula (XI) or (XII), and further, if desired, subjecting to alkylation and/or a method for salt-exchange. These reactions may be carried out by the same procedure as described hereinbefore.

Acid additional salts

Glycerol derivatives of the general formula (I), wherein $R^2$ represents an amino group or $R^3$ represents a group of general formula: $-N(R^6)_2$ (wherein $R^6$ is as defined hereinbefore.) may be converted into acid additional salts thereof. Preferably, acid additional salts are non-toxic salts and are water-soluble.

Suitable acid additional salts are, for example, inorganic acid salts such as hydrochloride, hydrobromide, hydroiodide, sulphate, phosphorate, nitrate, or organic acid salts such as acetate, lactate, tartrate, benzoate, citrate, methanesulphonate, ethanesulphonate, benzenesulphonate, toluenesulphonate or isethionate. Acid additional salts may be prepared by methods known *per se*, for example, reacting stoichiometric quantities of a compound of the general formula (I) wherein $R^2$ represents an amino group or $R^3$ represents a group of general formula: $-N(R^6)_2$ and a desirable acid in a suitable solvent.

Pharmacological activities

Glycerol derivatives of the general formula (I) and acid additional salts thereof have antagonistic effect on PAF, hypotensive effect like PAF, inhibitory effect on phospholipase and further, inhibitory effect growth of tumour cell, and are therefore, useful as a platelet aggregation inhibitor, anti-asthmatic agent, anti-allergic agent, anti-inflammatory agent, hypotensive agent and anti-tumour

agent. These effects were confirmed by standard laboratory test and, for example, inhibitory effect on PAF was confirmed by the following screen system.

Test method of inhibitory effect on PAF-induced platelet aggregation

Whole blood of male guinea pig was collected with a 3.8% aqueous solution of trisodium citrate in a proportion of 9:1 (v/v) and the mixture was centrifuged (120 x g) for 10 minutes at room temperature to obtain platelet rich plasma. Using obtained plasma, PAF (10 nM)-induced aggregation was determined by the absorbancy method using an aggrego-meter of Born [cf. J. Physiol., 162, 67 (1962)].

The evaluation is conducted using the concentration of compounds of the present invention required to produce a 50% inhibition of each effect, i.e. $IC_{50}$. The results are shown in the following table.

T a b l e I

| Example No. of tested compounds | Inhibitory effect on PAF-induced platelet aggregation ($IC_{50}$, M) |
|---|---|
| 2 | $5.0 \times 10^{-6}$ |
| 5 | $6.7 \times 10^{-6}$ |
| 9 | $3.7 \times 10^{-6}$ |
| 5(a) | $4.7 \times 10^{-6}$ |

Toxicity

On the other hand, it was confirmed that the acute toxicity of all extent of the compounds of the present invention was more than 100 mg/Kg by intravenous administration. Therefore, glycerol derivatives of the present invention may be considered to be sufficiently safe and suitable for medical use. For example, the mortality (daed/used) in glycerol derivatives of the present invention, i.e. compounds prepared in Example 4 was 0/5, when glycerol derivatives was intravenous administered to tail vein in five

mice at the dose of 100 mg/Kg.

Application for medical use

For the purpose hereinbefore described, glycerol derivatives of the general formula (I), or non-toxic acid additional salts thereof may normally be administered systemically or partially, usually by oral or parenteral administration. The doses to be administered is determined depending upon age, body weight, symptom, the desired therapeutic effect, the route of administration, and the duration of the treatment etc. In the human adult, the doses per person per dose are generally between 1 mg and 1 g, preferably between 20 mg and 200 mg by oral administration, up to several times per day, and between 100 µg and 100 mg, preferably between 1 mg and 10 mg by parenteral administration up to several times per day.

As mentioned above, the doses to be used depent on various conditions. Therefore, there are cases in which doses lower than or greater than the ranges specified above may be used.

Solid compositions according to the present invention for oral administration include compressed tablets, dispersible powders and granules. In such solid compositions, one or more of the active compound(s) is or are, admixed with at least one inert diluent such as lactose, mannitol, glucose, hydroxypropylcellulose, microcrystalline cellulose, starch, polyvinylpyrrolidone or magnesium metasilicate aluminate. The

compositions may also comprise, as is normal practice, additional substances other than inert diluents e.g. lubricating agents such as magnesium stearate, and, disintegrating agents such as cellulose calcium gluconate. The tablets or pills may, if desired, be made into gastric film-coated or enteric film-coated tablets or pills, such as sugar-coated, gelatin-coated, hydroxypropylcellulose-coated or hydroxypropylmethyl cellulose phthalate-coated tablets or pills; two or more of layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more of the active compound(s).

Preparations for injection according to the present invention for parenteral administration include sterile aqueous or non-aqueous solutions, suspensions or emulsions.

Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, POLYSORBATE 80 (resistered Trade Mark). These compositions may also include adjuvants such as preserving, wetting, emulsifying and despersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions for parenteral administration include liqiuds for external use, and endermic liniments such as ointments, suppositories for rectal administration and pessaries for vaginal administration which comprise one or more of the active compound(s) and may be prepared by known methods.

Preffered compounds

Preferred compounds included in the present invention of the general formula (I) are, for example; glycerol derivatives of general formula:

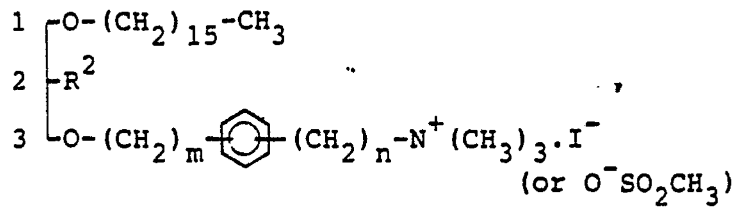 (Ih)

wherein $R^2$, m and n represent groups represented by the following ones in the table 2, respectively; and compounds of the general formula (Ih) wherein $R^2$ represents a group other than an amino group and the oxygen atom of the 3rd position [corresponding to the symbol A in the formula (I) ] of the formula (Ih) is replaced by a carbonyloxy group.

Table II

| Compound No. | $R^2$ | m | n |
|---|---|---|---|
| 1 | hydroxy | 3 | 0 |
| 2 | " | 1 | 3 |
| 3 | " | 1 | 4 |
| 4 | " | 0 | 3 |
| 5 | " | 0 | 4 |
| 6 | amino | 3 | 0 |
| 7 | " | 1 | 3 |
| 8 | " | 1 | 4 |
| 9 | " | 0 | 3 |
| 10 | " | 0 | 4 |
| 11 | azido | 3 | 0 |
| 12 | " | 1 | 3 |
| 13 | " | 1 | 4 |
| 14 | " | 0 | 3 |
| 15 | " | 0 | 4 |
| 16 | ethyl | 3 | 0 |
| 17 | " | 1 | 3 |
| 18 | " | 1 | 4 |

T a b l e II (continuation)

| Compound No. | $R^2$ | m | n |
|---|---|---|---|
| 19 | ethyl | 0 | 3 |
| 20 | " | 0 | 4 |
| 21 | ethoxy | 3 | 0 |
| 22 | " | 1 | 3 |
| 23 | " | 1 | 4 |
| 24 | " | 0 | 3 |
| 25 | " | 0 | 4 |
| 26 | benzyloxy | 3 | 0 |
| 27 | " | 1 | 3 |
| 28 | " | 1 | 4 |
| 29 | " | 0 | 3 |
| 30 | " | 0 | 4 |
| 31 | acetoxy | 3 | 0 |
| 32 | " | 1 | 3 |
| 33 | " | 1 | 4 |
| 34 | " | 0 | 3 |
| 35 | " | 0 | 4 |
| 36 | N-methylcarbamoyloxy | 3 | 0 |

Table II (continuation)

| Compound No. | $R^2$ | m | n |
|---|---|---|---|
| 37 | N-methylcarbamoyloxy | 1 | 3 |
| 38 | " | 1 | 4 |
| 39 | " | 0 | 3 |
| 40 | " | 0 | 4 |
| 41 | acetylamino | 3 | 0 |
| 42 | " | 1 | 3 |
| 43 | " | 1 | 4 |
| 44 | " | 0 | 3 |
| 45 | " | 0 | 4 |
| 46 | isobutoxycarbonylamino | 3 | 0 |
| 47 | " | 1 | 3 |
| 48 | " | 1 | 4 |
| 49 | " | 0 | 3 |
| 50 | " | 0 | 4 |
| 51 | N'-methylureido | 3 | 0 |
| 52 | " | 1 | 3 |
| 53 | " | 1 | 4 |
| 54 | " | 0 | 3 |
| 55 | " | 0 | 4 |

The following Reference Examples and Examples illustrates the present invention, but not limit the present invention. In the Reference Examples and Examples, "TLC", "NMR", "IR" and "MS" represent "Thin layer chromatography", "Nuclear magnetic resonance spectrum", "Infrared absorption spectrum" and "Mass spectrum", respectively. The ratios of the solvent used in chromatographic separations are by volume. The solvents in parentheses in "TLC" show the developing solvents. Unless otherwise specified, "IR" was measured by the liquid film method and "NMR" was measured in a deuterochloroform ($CDCl_3$) solution.

In the formula , symbols "Ms" and "THP" represent "methanesulphonyl (mesyl) group" and "tetrahyropyran-2-yl group", respectively.

Reference Example 1

Synthesis of

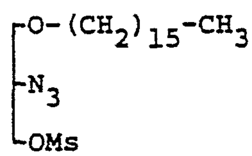

To a mixture of 2.37 g of (2RS)-1-O-hexadecyl-2-dehydroxy - 2-azidoglycerol ( compound described in

Reference Example 27 of the specification of European Patent Publication No. 109,255 ), 2 ml of triethylamine and 30 ml of methylene chloride, 0.7 ml of mesyl chloride was added, under ice-cooling. After stirring at the same temperature for 30 min, to the reaction mixture, 200 ml of diethyl ether was added. The diluted solution was washed with water and a saturated aqueous solution of sodium chloride, respectively, dried over anhydrous sodium sulphate and then concentrated under reduced pressure to give 2.85 g of the title compound haveing the following physical characteristic as crude product:

TLC (chloroform:acetone=10:1): Rf=0.75.

Reference Example 2

Synthesis of

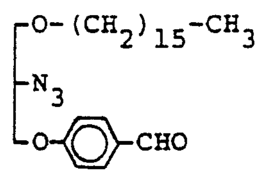

A mixture of 1 g of mesyloxy compound (prepared in Reference Example 1), 341 mg of p-hydroxybenzaldehyde, 3.1 g of potassium bicarbonate and 30 ml of acetone was refluxed for 2 days. To the reaction mixture, ethyl acetate was added, and the diluted solution was washed with

water, diluted hydrochloric acid, water, a saturated aqueous solution of sodium bicarbonate, water and a saturated aqueous solution of sodium chloride, respectively, dried over anhydrous sodium sulphate and then concentrated under reduced pressure. The residue was purified by column chromatography on silica gel (eluent: ethyl acetate: n-hexane=1:10) to give 411 mg of the title compound having the following physical characteristics:

TLC (ethyl acetate:n-hexane=1:5): Rf=0.39;

NMR: $\delta$= 9.91 (1H, s), 7.85 (2H, d), 7.03 (2H, d), 4.3 - 3.4 (7H, m), 1.8 - 1.0 (28H, m), 0.87 (3H, m);

IR: $\nu$= 2940, 2860, 2100, 1700, 1605, 1260, 1120 $cm^{-1}$;

MS: m/e= 445 ($M^+$), 417, 324, 282.

By the same procedure as Reference Example 2, the following compound was obtained:

(a)

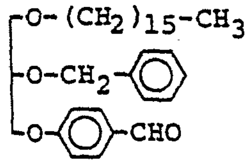

Starting material: (2RS)-1-O-hexadecyl-2-O-benzyl-3-O-

mesylglycerol (compound described in Reference Example 12 of the specification of European Patent Publication No. 109,255 );

TLC (ethyl acetate:n-hexane=1:5): Rf=0.42;

NMR: $\delta$ = 9.7 (1H, s), 7.68 (2H, d), 7.2 (5H, s), 6.86 (2H, d), 4.63 (2H, s), 4.2 − 3.2 (7H, m), 1.9 − 0.7 (31H, m);

IR : $\nu$ = 2930, 2860, 1700, 1600, 1110 $cm^{-1}$.

Reference Example 3

Synthesis of

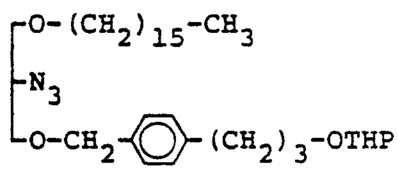

In an atmosphere of argon, a mixture of 245 mg of (2RS)-1-O-hexadecyl-2-dehydroxy-2-azidoglycerol ( compound described in Reference Example 27 of the specification of European Patent Publication No. 109,255 ), 54 mg of sodium hydride(content: 64.1%) and 4 ml of toluene was stirred for 30 min at 70°C. After cooling, to the reaction solution, 231 mg of 4-mesyloxymethyl-1-[3-(tetrahydropyran-2-yloxy)propyl]benzene dissolved in

4 ml of toluene was added with ice-cooling. The mixture was stirred overnight at 70°C. To the reaction mixture, water was added and then concentrated under reduced pressure. The residue was purified by column chromatography on silica gel (eluent: ethyl acetate:n-hexane=1:10) to give 198 mg of the title compound having the following physical characteristics:

TLC (ethyl acetate:n-hexane=1:10): Rf=0.56;

NMR: $\delta$ = 7.4 - 7.1 (4H, m)、4.64 - 4.46 (1H, m)
4.53 (2H, s)、4.1 - 3.20 (11H, m)、
2.72 (2H, t)、2.4 - 1.1 (36H, m)、
1.04 - 0.65 (3H, m) ;

IR: $\nu$ = 2920、2850、2100、1460、1120 $cm^{-1}$;

MS: m/e = 573 ($M^+$)、544、514、488、460。

By the same procedure as Reference Example 3, the following compound was obtained:

(a)

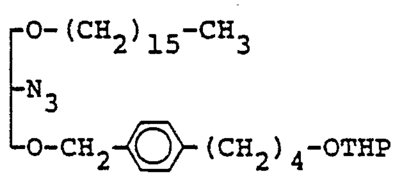

Starting material: (2RS)-1-O-hexadecyl-2-dehydroxy-2-azido-

glycerol (compound described in Reference Example 27 of the specification of European Patent Publication No. 109,255 ) and 4-mesyloxymethyl-1-[4-(tetrahydropyran-2-yloxy)butyl)]-benzene;

TLC (ethyl acetate:n-hexane=1:5): Rf=0.5;

NMR: δ= 7.0 (4H, bs), 4.6-4.3 (2H, m), 4.0-3.1 (11H, m), 2.83-2.3 (2H, m), 2.0-0.7 (41H, m);

IR: ν= 2930, 2850, 2100, 1460, 1120 $cm^{-1}$;

MS: m/e= 559, 528, 502, 474.

Reference Example 4

Synthesis of

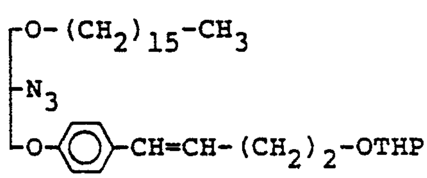

In an atmosphere of argon, to a solution of 328 mg of 3-(tetrahydropyran-2-yloxy)propyltriphenylphosphonium bromide in 10 ml of dry tetrahydrofuran, 0.45 ml of a 1.35 M solution of n-butyl lithium in n-hexane under ice-cooling. After stirring for 30 min at the same temperature, to the reaction solution, 200 mg of benzaldehyde compound (prepared in Reference Example 2) dissolved in 5 ml of dry tetrahydrofuran was added under ice-cooling. The solution was stirred for 30 min at the same temperature and for 1 hr at room temperature. The reaction mixture was poured into water, and the mixture was extracted with ethyl acetate. The extract was washed with water and a saturated aqueous solution of sodium chloride, succesively, dried over anhydrous sodium sulphate and then concentrated under reduced pressure. The residue was purified by column chromatography on silica gel (eluent: ethyl acetate:n-hexane=1:10) to give 226 mg of the title compound having the following physical characteristics:

TLC (ethyl acetate:n-hexane=1:5): Rf=0.53 & 0.57;

NMR: $\delta$= 7.4-6.7 (4H, m)、6.5-5.4 (2H, m)、4.7-4.5 (1H, m)、4.26-3.34 (11H, m)、2.80-2.36 (2H, m)、1.98-1.0 (34H, m)、0.86 (3H, m) ;

IR: $\nu$= 2940、2860、2100、1610、1520、1250、1120 $cm^{-1}$ ;

MS: m/e= 571 ($M^+$)、543、487、469。

By the same procedure as Reference Example 4, the following compound was obtained:

(a)

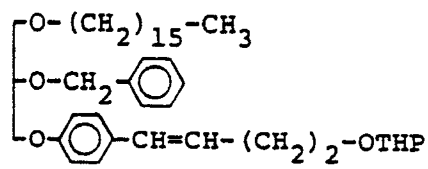

Starting material: benzaldehyde compound prepared in Reference Example 2(a);

TLC (ethyl acetate:n-hexane=1:5): Rf=0.54 & 0.59;

NMR: $\delta$= 7.1 (5H, s), 7.2 - 6.0 (2H, m), 4.6 (2H, s), 4.7 - 4.4 (1H, m), 4.1 - 3.2 (11H, m), 2.8 - 2.2 (2H, m), 1.9 - 0.7 (39H, m) ;

IR: $\nu$= 2930, 2850, 1600, 1120 $cm^{-1}$;

MS: m/e= 636 ($M^+$), 552, 534 .

Reference Example 5

Synthesis of

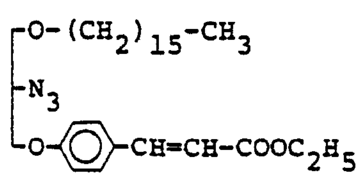

In an atmosphere of argon, a mixture of 25 mg of

sodium hydride, 151 mg of diethylethoxycarbonylmethyl phosphonate and 4 ml of dry tetrahydrofuran was stirred for 10 min at room temperature. To the reaction mixture, 200 mg of benzaldehyde compound (prepared in Reference Example 2) dissolved in 2 ml of dry tetrahydrofuran was added. After stirring for 15 min at room temperature, the reaction mixture was poured into diluted hydrochloric acid. The mixture was extracted with ethyl acetate, and the extract was washed with water and a saturated aqueous solution of sodium chloride, succesively, dried over anhydrous magnesium sulphate and then concentrated under reduced pressure to give 208 mg of the title compound having the following physical characteristics as crude product:

TLC (ethyl acetate:n-hexane=1:5): Rf=0.48;

NMR: $\delta$= 7.66 (1H, d), 7.50 (2H, d), 6.96 (2H, d), 6.36 (1H, d), 4.7-3.4 (9H, m), 2.0-0.7 (34H, m);

MS: m/e= 515 ($M^+$), 487.

Reference Example 6

Synthesis of

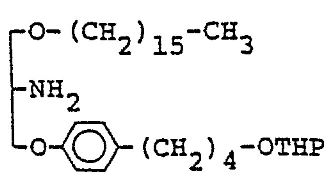

In an atmosphere of hydrogen, a mixture of

172 mg of azido compound (prepared in Reference Example 4), 80 mg of palladium-carbon (content:10%) and 4 ml of ethanol was stirred for 2 hrs at room temperature. The catalyst was removed off by filtration, the filtrate was concentrated under reduced pressure to give 200 mg of the title compound having the following physical characteristics as crude product:

TLC (chloroform:methanol=10:1): Rf=0.46;

NMR: $\delta$= 6.95 (4H, dd), 4.64 - 4.5 (1H, m), 4.1 - 3.2 (10H, m), 2.2 - 1.0 (40H, m) 1.0 - 0.7 (3H, m) ;

IR: $\nu$ = 2920, 2850, 1510, 1460, 1110 $cm^{-1}$ ;

MS: m/e = 547 ($M^+$), 489, 463 .

Reference Example 7

Synthesis of

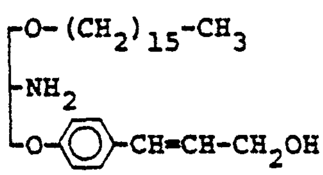

A mixture of 208 mg of azido compound (prepared in Reference Example 5) and 200 mg of lithium aluminium hydride and 10 ml of diethyl ether was refluxed for 3 hrs.

To the reaction solution, a 10% aqueous solution of sodium hydroxide was added for quenching unreacted reducing agent. The mixture was dried over anhydrous magnesium sulphate and filtered. The filtrate was concentrated under reduced pressure to give 149 mg of the title compound having the following physical characteristics:

TLC (chloroform:methanol=10:1): Rf=0.50;

NMR: δ = 7.49 - 6.72 (4H, m), 6.54 (1H, d), 6.19 (1H, dt), 4.4 - 3.2 (9H, m), 1.9 - 1.0 (28H, m), 0.84 (3H, m);

MS: m/e = 447 ($M^+$), 298, 284.

By the same procedure as Reference Example 7, the following compounds were obtained:

(a)

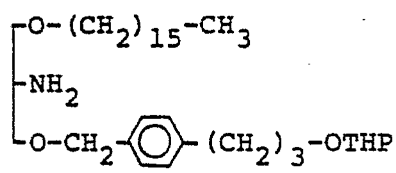

Starting material: azido compound prepared in Reference Example 3;

TLC (chloroform:methanol=10:1): Rf=0.59;

MS: m/e = 547 ($M^+$), 462.

(b)

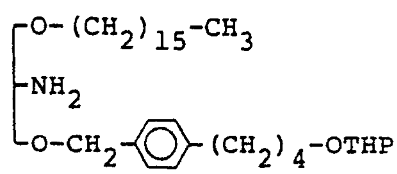

Starting material: azido compound prepared in Reference Example 3(a);

TLC (chloroform:acetone=10:1): Rf=0.10;

NMR: $\delta$= 7.3 - 7.0 ( 4H, m ), 4.63 - 4.3 ( 3H, m ), 4.0 - 3.0 ( 10H, m ), 2.8 - 2.4 ( 3H, m ), 2.0 - 0.7 ( 43H, m ) ;

IR: $\nu$= 2940, 2850, 1460, 1120cm$^{-1}$;

MS: m/e= 561 ( $M^+$ ), 476, 460.

Reference Example 8

Synthesis of

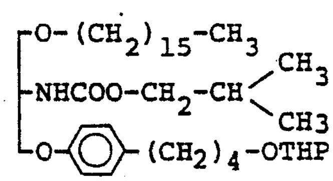

To a mixture of 200 mg of amino compound (prepared in Reference Example 6), 4 ml of methylene chloride and 0.2 ml of triethylamine, 62 µl of isobutyl chloroformate was added with ice-cooling. The mixture was stirred for 15 min at room temperature. The reaction mixture was

diluted with 30 ml of methylene chloride. The diluted solution was washed with water, dried over anhydrous sodium sulphate and then concentrated under reduced pressure to give 170 mg of the title compound having the following physical characteristics:

TLC (ethyl acetate:n-hexane=1:5): Rf=0.33;

NMR: $\delta$ = 6.8 (4H, dd), 5.3 - 4.9 (1H, m), 4.76 - 4.46 (1H, m), 4.3 - 3.1 (14H, m), 2.9 - 2.3 (3H, m), 2.1 - 0.8 (48H, m);

MS: m/e = 647 ($M^+$), 563, 489.

By the same procedure as Reference Example 8, the following compounds were obtained:

(a)

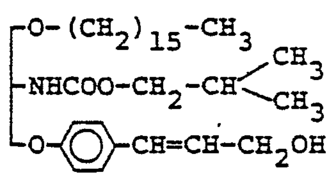

Starting material: amino compound prepared in Reference Example 7;

TLC (chloroform:acetone=10:1): Rf=0.43;

NMR: $\delta$ = 7.2 - 5.7 (6H, m), 4.2 - 3.0 (11H, m) 2.1 - 0.6 (38H, m);

IR: $\nu$ = 3400, 2930, 2860, 1720, 1510 $cm^{-1}$;

MS: m/e = 547 ($M^+$), 473, 446.

(b)

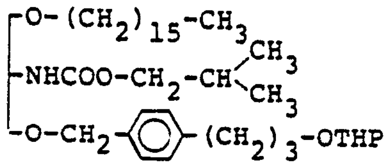

Starting material: amino compound prepared in Reference Example 7(a);

TLC (ethyl acetata:n-hexane=1:5): Rf=0.26;

NMR: δ= 7.3-6.9 (4H, m)、5.1-4.8 (1H, m)、
4.7-4.4 (3H, m)、4.1-3.2 (13H, m);

IR: ν= 2920、2850、1720、1460、
1120 $cm^{-1}$;

MS: m/e= 647 ($M^+$)、573、562.

(c)

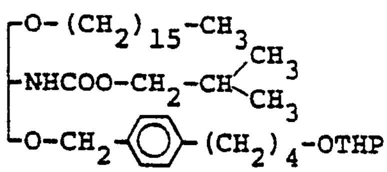

Starting material: amino compound prepared in Reference Example 7(b);

TLC (ethyl acetate:n-hexane=1:5): Rf=0.33;

NMR: δ= 7.1 (4H, bs)、5.1-4.8 (1H, m)、4.7
-4.3 (3H, m)、4.1-3.1 (13H, m)、
2.9-2.4 (2H, m)、2.0-0.7 (48H, m);

MS: m/e= 604、588、576.

Reference Example 9

Synthesis of

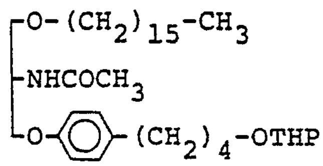

A mixture of 75 mg of amino compound (prepared in Reference Example 6), 0.1 ml of acetic anhydride, 0.5 ml of triethylamine and 5 ml of methylene chloride was stirred for 1 hr at room temperature. The reaction mixture was concentrated under reduced pressure to give 80 mg of the title compound having the following physical characteristics as crude product:

TLC (choloform:methanol=10:1): Rf=0.86;

NMR: $\delta$ = 6.98 (2H, d), 6.68 (2H, d), 6.3-6.0 (1H, m), 4.6-4.4 (1H, m), 4.1-3.0 (11H, m), 2.7-2.3 (2H, m), 2.1 (3H, s), 2.0-0.7 (41H, m) ;

MS: m/e = 589 ($M^+$), 505, 446.

Reference Example 10

Synthesis of

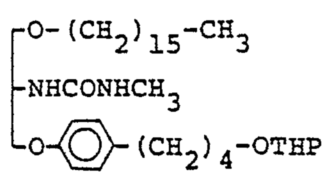

A mixture of 71 mg of amino compound (prepared in Reference Example 6), 15 μl of methyl isocyanate, 0.1 ml of triethylamine and 5 ml of methylene chloride was stirred for 1 hr at room temperature. The reaction mixture was concentrated under reduced pressure to give 80 mg of the title compound having the following physical characteristics as crude product:

TLC (chloroform:methanol=10:1): Rf=0.69;

NMR: $\delta$ = 6.96 (2H, d), 6.70 (2H, d), 5.4-5.0 (2H, m), 4.2-3.0 (11H, m), 2.7 (3H, d), 2.8-2.3 (2H, m), 2.0-0.6 (41H, m).

Reference Example 11

Synthesis of

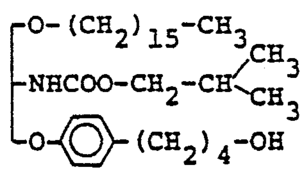

A mixture of 170 mg of tetrahydrofuran-2-yloxy compound (prepared in Reference Example 8), about 5 mg of p-toluenesulphonic acid, 5 ml of methanol and 2 ml of methylene chloride was stirred for 2 hrs at room temperature. To the reaction mixture, a saturated aqueous solution of

sodium bicarbonate was added, dried over anhydrous sodium sulphate and then concentrated under reduced pressure. The residue obtained was purified by column chromatography on silica gel (eluent: chloroform: methanol=30:1) to give 163 mg of the title compound having the following physical characteristics:

TLC (chloroform:acetone=10:1): Rf=0.50;

NMR: $\delta$= 6.85 (4H, dd), 5.3-4.9 (1H, m), 4.3-3.3 (12H, m), 2.83-2.4 (3H, m), 2.0-0.7 (42H, m) ;

IR: $\nu$= 3400, 2930, 2860, 1710, 1510, 1110 $cm^{-1}$;

MS: m/e= 563($M^+$), 548, 489, 462 .

By the same procedure as Reference Example 11, the following compounds were obtained:

## Table 1.

| Reference Example No. | Starting material | Formula of the product | Physical characteristics of the product: TLC (Rf value; developer) | NMR (δ) | IR (ν) | MS (m/e) |
|---|---|---|---|---|---|---|
| 11 (a) | compound of Reference Example 8 (b) | 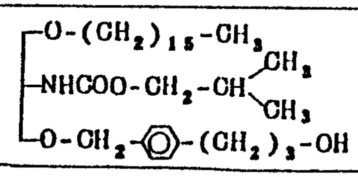 | 0.44 (chloroform: acetone=10:1) | 7.1 (4H, s), 5.1 - 4.8 (1H, m), 4.53 (2H, s), 4.0 - 3.2 (11H, m), 2.9 - 2.5 (2H, m), 2.1 - 1.1 (31H, m) | 3400, 2930, 2850, 1700, 1460, 1110 | 563 ($M^+$), 489, 399 |
| 11 (b) | compound of Reference Example 8 (c) | 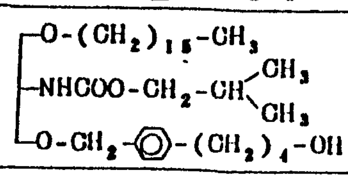 | 0.39 (chloroform: acetone=10:1) | 7.1 (4H, bs), 5.2 - 4.8 (1H, m), 4.43 (2H, s), 4.0 - 3.2 (11H, m), 2.8 - 2.4 (2H, m), 2.0 - 0.7 (43H, m) | 3400, 2940, 2860, 1720, 1460 | |
| 11 (c) | compound of Reference Example 9 | 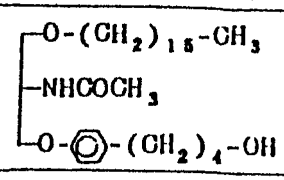 | 0.58 (chloroform: acetone=10:1) | | | |
| 11 (d) | compound of Reference Example 10 | 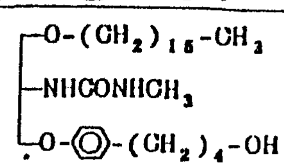 | 0.46 (chloroform: acetone=10:1) | | | 520 ($M^+$), 489 |
| 11 (e) | compound of Reference Example 4 (a) | 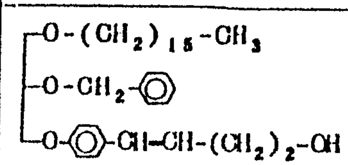 | 0.13 (ethyl acetate: n-hexane=1:5) | 7.2 (5H, s), 7.2 - 6.0 (2H, m), 4.66 (2H, s), 4.2 - 3.3 (9H, m), 2.8 - 2.3 (2H, m), 2.0 - 0.7 (31H, m). | 3400, 2930, 2850, 1600, 1460, 1110 | |

Reference Example 12

Synthesis of

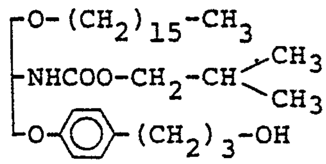

By the same procedure as Reference Example 6, with hydroxypropyl compound [Reference Example 8(a)], the title compound having the following physical characteristics:

TLC (chloroform:acetone=10:1): Rf=0.44;

[ a silica gel plate pre-treated with silver nitrate was used.]

MS: m/e=549($M^+$)、475、398,

Reference Example 13

Synthesis of

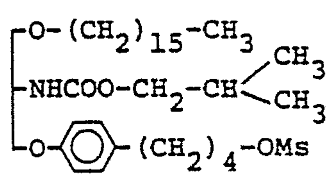

To a mixture of 163 mg of hydroxy compound (prepared in Reference Example 11), 0.15 ml of triethylamine and 2 ml of methylene chloride, 27 µl of mesyl chloride was added under ice-cooling. The mixture was

stirred for 15 min at the same temperature. To the reaction solution, 30 ml of ethyl acetate was added. The diluted solution was washed with water and a saturated aqueous solution of sodium chloride, succesively, dried over anhydrous sodium sulphate and then concentrated under reduced pressure to give 180 mg of the title compound having the following physical characteristic:

TLC (chloroform:acetone=10:1): Rf=0.72.

By the same procedure as Reference Example 13, the following compounds were obtained:

Table 2.

| Reference Example No. | Starting material | Formula of the product | TLC (Rf value; developer) |
|---|---|---|---|
| 13 (*a*) | compound of Reference Example 12 | 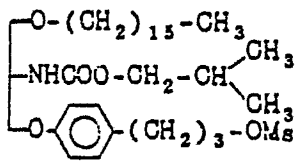 | 0.79 (chloroform: acetone=10:1) |
| 13 (*b*) | compound of Reference Example 11(a) | 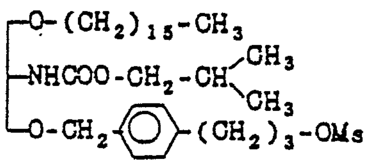 | 0.73 (chloroform: acetone=10:1) |
| 13 (*c*) | compound of Reference Example 11(b) | 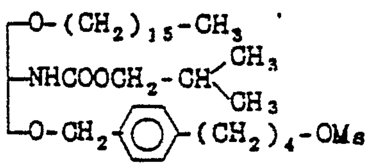 | 0.66 (chloroform: acetone=10:1) |
| 13 (*d*) | compound of Reference Example 11(c) | 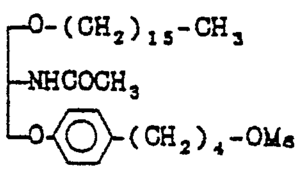 | 0.78 (chloroform: acetone=10:1) |
| 13 (*e*) | compound of Reference Example 11(d) | 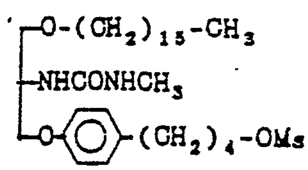 | 0.62 (chloroform: acetone=10:1) |
| 13 (*f*) | compound of Reference Example 11(e) | 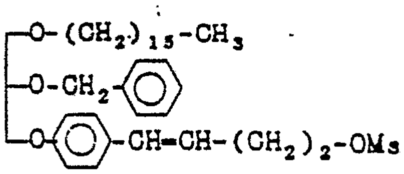 | 0.87 (chloroform: acetone=10:1) |

Reference Example 14

Synthesis of

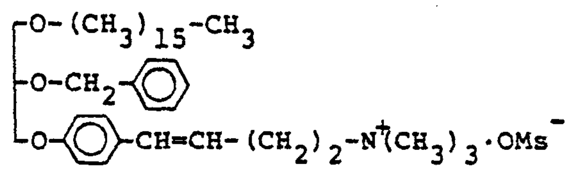

A mixture of 269 mg of mesyloxy compound [prepared in Reference Example 13(f)], 0.3 ml of a 30% aqueous solution of trimethylamine and 10 ml of dimethylformamide was stirred overnight at 60°C. The reaction solution was concentrated under reduced pressure. The residue was purified by column chromatography on silica gel (eluent: chloroform:methanol=10:1 → chloroform:methanol:water=65:35:2) to give 199 mg of the title compound having the following physical characteristics:

TLC (ethyl acetate:acetic acid:water=3:1:1):

Rf=0.60 & 0.61;

NMR: $\delta$ =7.2(5H, s), 7.2-6.0(2H, m), 4.7(2H, s), 4.2-3.1(9H, m), 3.33 & 3.23(9H, s), 2.7(3H, s), 2.9-2.3(2H, m), 1.9-0.7 (31H, m).

Example 1

Synthesis of

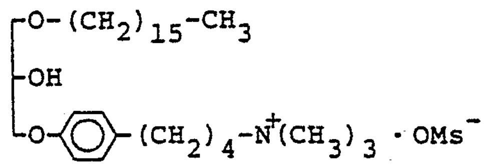

By the same procedure as Reference Example 6, with benzyloxy compound (prepared in Reference Example 14), the title compound having the following physical characteristics:

TLC (ethyl acetate:acetic acid:water=3:1:1):

Rf=0.54;

NMR: $\delta$= 6.81 (4H, q)、4.1 - 3.0 (9H, m)、3.1 (9H, s)、2.61 (3H, s)、2.7 - 2.2 (2H, m)、2.0 - 0.6 (35H, m);

IR: $\nu$= 3350、2910、2850、1600、1500、1460、1120 $cm^{-1}$.

Example 2

Synthesis of

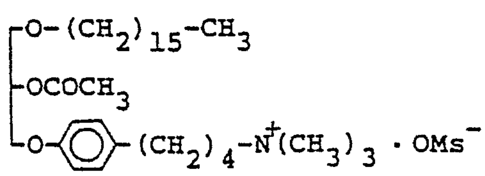

A mixture of 174 mg of hydroxy compound (prepared in Example 1), 0.6 ml of acetic anhydride and 5 ml of

pyridine was stirred overnight at 40°C. The reaction mixture was concentrated under reduced pressure. The residue was purified by column chromatogrphy on silica gel (eluent: chloroforlm:methanol=10:1 ⟶ chloroform: methanol:water=65:35:2) to give 85 mg of the title compound having the following physical characteritics:

melting point: 36-40°C;

TLC (ethyl acetate:acetic acid:water=3:1:1):

Rf=0.60;

NMR: δ= 7.06 (2H, d)、6.82 (2H, d)、5.30 (1H, m)、4.16-4.05 (2H, m)、3.65 (2H, d)、3.59-3.4 (4H, m)、3.30 (9H, s)、2.75 (3H, bs)、2.70-2.58 (2H, m)、2.08 (3H, s)、2.10-1.45 (6H, m)、1.25 (26H, m)、0.88 (3H, m) ;

IR: ν= 3400、2920、2850、1740、1510、1120 $cm^{-1}$;

MS: m/e= 533、490、474.

Example 3

Synthesis of

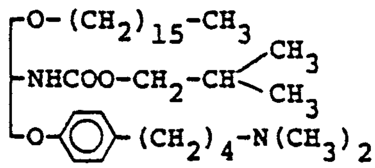

A mixture of 180 mg of mesyloxy compound (prepared in Reference Example 13), 1 ml of a 40% aqueous solution of dimethylamine and 5 ml of dimethylformamide was stirred overnight at 60°C. The reacetion mixture was concentrated under reduced pressure, and the residue obtained was purified by column chromatography on silica gel (elueInt: chcloform:methanol:triethylamine=30:1:0.05) to give 139 mg of the title compound having the following physical characteristics:

TLC (ethyl acetate:acetic acid:water=3:1:1):

Rf=0.67;

NMR: δ= 7.07 (2H, d), 6.82 (2H, d), 5.20-5.05 (1H, m), 4.18-3.9 (3H, m), 3.85 (2H, d), 3.72-3.44 (2H, m), 3.43 (2H, t), 2.56 (2H, t), 2.40-2.24 (2H, m), 2.23 (6H, s), 2.1-1.40 (7H, m), 1.26 (26H, m), 0.92 (6H, d), 0.88 (3H, m) ;

IR: ν= 2930, 2850, 1720, 1510, 1110 $cm^{-1}$;

MS: m/e= 590 ($M^+$), 575, 547, 533.

By the same procedure as Example 3, the following compounds were obtained:

Table 3.

| Example No. | Starting material | Formula of the product | Physical characteristics of the product | | | |
|---|---|---|---|---|---|---|
| | | | TLC (Rf value; developer) | NMR (δ) | IR (ν) | MS (m/e) |
| 3 (a) | compound of Reference Example 13(a) | 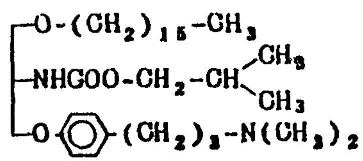 | 0.62 (ethyl acetate: acetic acid: water=3:1:1) | 7.07(2H,d), 6.82(2H,d), 5.20-5.05(1H,m), 4.20-3.94(3H,m), 3.85(2H,d), 3.72-3.48(2H,m), 3.42(2H,t), 2.58(2H,t), 2.40-2.24(2H,m), 2.25(6H,s), 2.00-1.45(5H,m), 1.26(26H,m), 0.92(6H,d), 0.88(3H,m) | 2930, 2850, 1720, 1510, 1110 | 576($M^+$), 503 |
| 3 (b) | compound of Reference Example 13(b) | 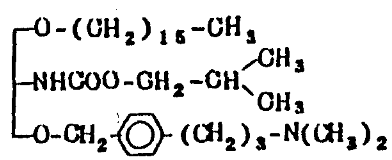 | 0.65 (ethyl acetate: acetic acid: water=3:1:1) | 7.18(4H,dd), 5.10-4.98(1H,m), 4.48(2H,s), 4.05-3.85(1H,m), 3.83(2H,d), 3.70-3.35(6H,m), 2.64(2H,t), 2.30-2.25(2H,m), 2.24(6H,s) 2.00-1.45(5H,m), 1.26(26H,m), 0.92(6H,d), 0.88(3H,m) | 2940, 2860, 1730, 1460, 1110 | 590($M^+$), 548 |
| 3 (c) | compound of Reference Example 13(c) | 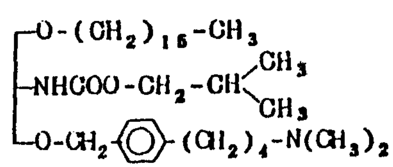 | 0.67 (ethyl acetate: acetic acid: water=3:1:1) | 7.16(4H,q), 5.10-4.96(1H,m), 4.47(2H,s), 3.83(2H,d), 4.00-3.86(1H,m), 3.65-3.35(6H,m), 2.62(2H,t), 2.24(6H,s), 2.40-2.20(2H,m), 2.00-1.44(7H,m), 1.26(26H,m), 0.91(6H,d), 0.88(3H,m) | 2930, 2860, 1725, 1460, 1110 | 604($M^+$), 589, 530 |

Example 4

Synthesis of

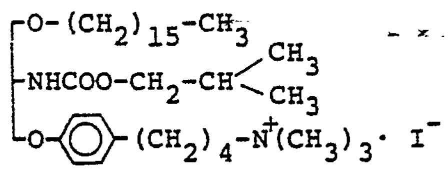

A mixture of 70 mg of dimethylamino compound (prepared in Example 3), 0.2 ml of methyl iodide, 3 ml of methanol and about 10 mg of potassium bicarbonate was stirred for 1 hr at room temperature. The reaction mixture was concentrated under reduced pressure. The residue was dissolved into little amount of ethyl acetate and the solution was filtered off to remove insoluble matter. The filtrate was concentrated under reduced pressure to give 76 mg of the title compound having the following physical characteristics:

TLC (ethyl acetate:acetic acid:water=3:1:1):

Rf=0.52;

NMR: δ= 7.08 (2H, d), 6.84 (2H, d), 5.21-4.06 (1H, m), 4.17-3.91 (3H, m), 3.85 (2H, d), 3.73-3.4 (6H, m), 3.40 (9H, s), 2.75-2.6 (2H, m), 2.00-1.44 (7H, m), 1.26 (26H, m), 0.93 (6H, d), 0.88 (3H, m) ;

IR: ν = 3450、2920、2850、1710、1510、
1110 $cm^{-1}$;

MS: m/e = 590、575、547、517 .

By the same procedure as Example 4, the following compounds were obtained:

Table 4.

| Example No. | Starting material | Formula of the product | Physical characteristics of the product | | | |
|---|---|---|---|---|---|---|
| | | | TLC (Rf value; developer) | NMR (δ) | IR (ν) | MS (m/e) |
| 4 (a) | compound of Example 3(a) | 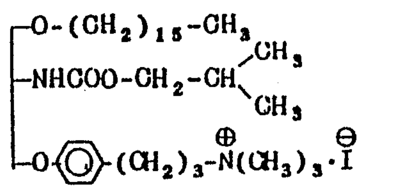 | 0.57 (ethyl acetate: acetic acid: water=3:1:1) | 7.22(2H, d), 6.86(2H, d) 5.22-5.06(1H, m), 4.20-3.92(3H, m), 3.86(2H, d) 3.70-3.30(6H, m), 3.40 (9H, s), 2.73(2H, t), 2.00-1.45(7H, m), 1.26 (26H, m), 0.93(6H, d), 0.88(3H, m) | 3450, 2920, 2850, 1700, 1505, 1460, 1110 | 585, 576 502 |
| 4 (b) | compound of Example 3(b) | 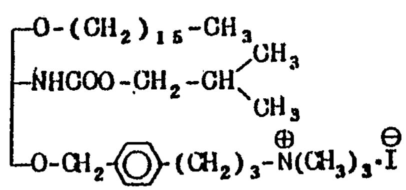 | 0.45 (ethyl acetate: acetic acid: water=3:1:1) | 7.22(4H, dd), 5.10-4.97 (1H, m), 4.48(2H, s), 4.02-3.85(1H, m), 3.82 (2H, d), 3.70-3.40(8H, m), 3.40(9H, s), 2.78 (2H, t), 2.23-2.04(2H, m), 1.98-1.80(1H, m), 1.70-1.46(2H, m), 1.26 (26H, m), 0.91(6H, d), 0.87(3H, m) | 3400, 2940, 2860, 1710, 1460, 1110 | 590, 575 547 |
| 4 (c) | compound of Example 3(c) | 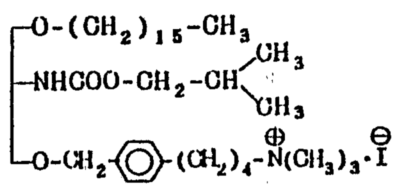 | 0.56 (ethyl acetate: acetic acid: water=3:1:1) melting point: 45-55°C | 7.19(4H, q), 5.10-4.94 (1H, m), 4.48(2H, s), 3.82(2H, d), 4.00-3.84 (1H, m), 3.39(9H, s), 3.72-3.35(8H, m), 2.80 -2.66(2H, bs), 2.00-1.45(7H, m), 1.26(26H, m), 0.92(6H, d), 0.88 (3H, m) | 3330, 2920, 2850, 1720, 1465, 1110 | 604, 589, 530 |

Example 5

Synthesis of

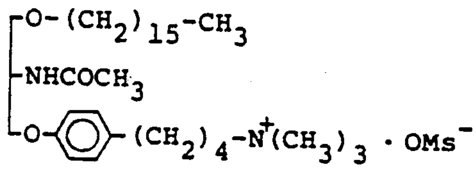

By the same procedure as Reference Example 14, with mesyloxy compound [prepared in Reference Example 13], the title compound having the following physical characteristics:

TLC (ethyl acetate:acetic acid:water=3:1:1):

Rf=0.50;

NMR: δ= 7.06 (2H, d)、6.84 (2H, d)、6.05-5.9 (1H, m)、4.5-4.25 (1H, m)、4.15-3.9 (2H, m)、3.7-3.3 (6H, m)、3.32 (9H, s)、3.0-2.5 (5H, m)、2.0 (3H, s)、2.2-1.4 (6H, m)、1.25 (26H, m)、0.88 (3H, m) ;

IR: ν= 3300、2920、2850、1640、1510、1120 $cm^{-1}$;

MS: m/e = 532、517、489 .

By the same procedure as Example 5, the following compound was obtained:

(a)

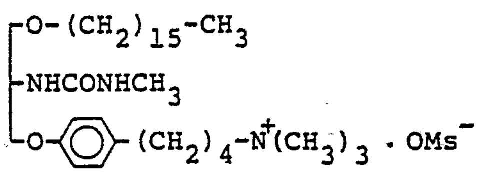

Starting material: mesyloxy compound prepared in Reference Example 13(e);

TLC (ethyl acetate:acetic acid:water=3:1:1): Rf=0.5 ;

NMR: δ= 7.04 (2H, d)、6.82 (2H, d)、5.4-5.1 (2H, m)、4.25-3.9 (3H, m)、3.7-3.2 (6H, m)、3.20 (9H, s)、2.85-2.5 (8H, m)、2.1-1.44 (6H, m)、1.24 (26H, m)、0.87 (3H, m);

IR: ν= 3360、2930、2850、1630、1510、1120 $cm^{-1}$;

MS: m/e= 547、532、516 .

Example 6

Synthesis of

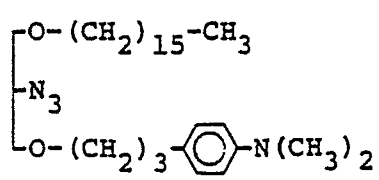

By the same procedure as Reference Example 3,

with (2RS)-1-O-hexadecyl-2-dehydroxy-2-azidoglycerol (compound described in Reference Example 27 of the specification of European Patent Publication No. 109,255 ) and p-(3-mesyloxypropyl)-N,N-dimethylaniline, the title compound having the following physical characteristics:

TLC (ethyl acetate:n-hexane=1:5): Rf=0.55;

NMR: $\delta$= 6.93 (2H, d), 6.56 (2H, d), 3.6-3.2 (9H, m), 2.83 (6H, s), 2.9-2.3 (2H, m), 2.2-0.7 (33H, m)

IR: $\nu$= 2930, 2850, 2100, 1620, 1520, 1120 $cm^{-1}$;

MS: m/e= 502 ($M^+$), 474.

Example 7

Synthesis of

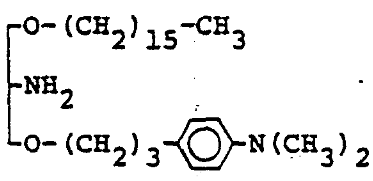

By the same procedure as Reference Example 7, with azido compound (prepared in Reference Example 6), the title compound having the following physical characteristics:

TLC (chloroform:methanol=10:1): Rf=0.45;

MS: m/e= 476($M^+$)、284 .

<u>Example 8</u>

Synthesis of

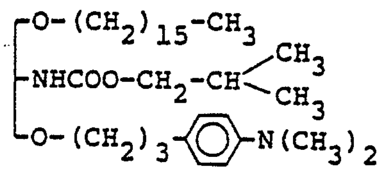

By the same procedure as Reference Example 8, with amino compound (prepared in Example 7), the title compound having the following physical characteristics:

TLC (ethyl acetate:n-hexane=1:5): Rf=0.45;

NMR: δ= 7.04 (2H, d)、6.68 (2H, d)、5.10-4.97 (1H, m)、3.96-3.80 (3H, m)、3.60-3.35 (8H, m)、2.90 (6H, s)、2.58 (2H, t)、2.00-1.45 (5H, m)、1.26 (26H, m)、0.92 (6H, d)、0.88 (3H, m) ;

IR: ν= 2930、2850、1720、1520、1110 $cm^{-1}$;

MS: m/e= 576($M^+$)、502、475、459.

Example 9

Synthesis of

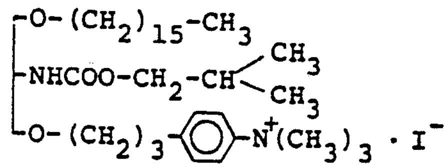

By the same procedure as Example 4, with dimethylamino compound (prepared in Example 8), the title compound having the following physical characteristics:

melting point: 50-63°C;

TLC (ethyl acetate:acetic acid:water=3:1:1): Rf=0.62;

NMR: $\delta$= 7.78 (2H, d), 7.40 (2H, d), 5.10-4.92 (1H, m), 3.99 (9H, s), 3.83-(2H, d), 3.92-3.78 (1H, m), 3.59-3.37 (8H, m), 2.75 (2H, t), 2.00-1.80 (3H, m), 1.70-1.46 (2H, m), 1.25 (26H, m), 0.92 (6H, d), 0.88 (3H, m);

IR(KBr tablet method): $\nu$=3450, 2930, 2850, 1710, 1510, 1470, 1120 $cm^{-1}$;

MS: m/e= 576, 502, 475, 459.

Example 10

5 g of (2RS)-1-O-hexadecyl-2-dehydroxy-2-isobutyloxycarbonylamino-3-O-[3-[4-(N,N,N-trimethylammonio)phenyl]propyl]glycerol iodide, 200 mg of cellulose calcium gluconate (disintegrating agent), 100 mg of magnesium stearate (lubricating agent) and 9.7 g of crystalline cellulose were admixed and punched out in conventional manner to give tablets each containing 50 mg of the active ingredient.

CLAIMS:

1. A glycerol derivative of general formula:

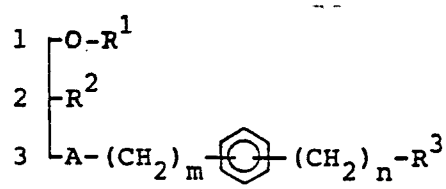

(I)

[wherein A represents an oxygen atom or a carbonyloxy group (wherein the carbonyl group should be attached to the group represented by $(CH_2)_m$ and the oxa group should be attached to the carbon atom at the 3rd position of the glycerol skelton), $R^1$ represents an alkyl group of 6 to 22 carbon atoms or an alkyl group of 2 to 5 carbon atoms being substituted by a phenyl group, $R^2$ represents a hydroxy group, an amino group , an azido group, an alkyl group of 1 to 20 carbon atom(s), an alkoxy group of 1 to 20 carbon atom(s) or a group of general formula:

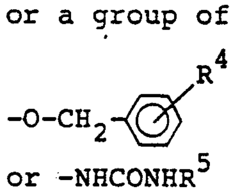 , $-OCOR^5$, $-OCONHR^5$, $-NHCOR^5$, $-NHCOOR^5$ or $-NHCONHR^5$ (wherein $R^4$ represents a hydrogen atom, a halogen atom, an alkyl group of 1 to 4 carbon atom(s) or an alkoxy group of 1 to 4 carbon atom(s) and $R^5$ represents an alkyl group of 1 to 10 carbon atom(s) or a phenyl group), $R^3$ represents a group of general formula: $-N(R^6)_2$ or $-N^+(R^6)_3.Y^-$ (wherein each $R^6$ represents an alkyl

group of 1 to 8 carbon atom(s) and which may be same or different to each other, or represents a heterocyclic ring of 4 to 10 ring members combining with 2 or 3 of $R^6$ and the nitrogen atom and if another $R^6$ not related to the heterocyclic ring exist, the $R^6$ represents an alkyl group of 1 to 8 carbon atom(s), and $Y^-$ represents an anion of acid.), m and n represent zero or an integer of from 1 to 6, respectively, and total number of m plus n should be more than 1. With the proviso that when A represents a carbonyloxy group, $R^2$ does not represent an amino group.]
or an acid additional salt thereof.

2. A compound according to claim 1 wherein $R^1$ represents an alkyl group of 14 to 18 carbon atoms.

3. A compound according to claim 2 wherein $R^1$ represents a hexadecyl group.

4. A compound according to claim of from 1 to 3 wherein $R^2$ represents a hydroxy group, an amino group, an azido group, an alkyl group of 1 to 6 carbon atom(s), an alkoxy group of 1 to 6 carbon atom(s) or a group of general formula:

$-O-CH_2-$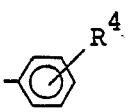, $-OCOR^5$, $-OCONHR^5$, $-NHCOR^5$, $-NHCOOR^5$ or $-NHCONHR^5$ (wherein $R^4$ represents a hydrogen atom, a chlorine atom, a methyl group or a methoxy group and $R^5$ represents an alkyl group of 1 to 6 carbon atom(s) or a phenyl

group).

5. A compound according to claim 4 wherein $R^2$ represents a hydroxy group, an amino group, an azido group or a group of general formula: $-OCOR^5$, $-NHCOR^5$, $-NHCOOR^5$ or $-NHCONHR^5$ (wherein $R^5$ represents an alkyl group of 1 to 6 carbon atom(s) or a phenyl group).

6. A compound according to a claim of from 1 to 5 wherein A represents an oxygen atom.

7. A compound according to a claim of from 1 to 5 wherein A represents a carbonyloxy group.

8. A compound according to a claim of from 1 to 7 wherein $R^3$ represents a group of general formula:
$-N(R^6)_2$ or $-N^+(R^6)_3.Y^-$ (wherein each $R^6$
represents an alkyl group of 1 to 4 carbon atom(s) and which may be same or different to each other, and $Y^-$ represents an anion of acid.

9. A compound according to claim 1 which is:
(2RS)-1-hexadecyl-3-O-[4-[4-(N,N,N-trimethylammonio)-butyl]phenyl]glycerol mesylate,
(2RS)-1-O-hexadecyl-2-dehydroxy-2-amino-3-O-[3-[4-(N,N-dimethylamino)phenyl]propyl]glycerol,
(2RS)-1-O-hexadecyl-2-dehydroxy-2-azido-3-O-[3-[4-(N,N-dimethylamino)phenyl]propyl]glycerol,

(2RS)-1-O-hexadecyl-2-O-acetyl-3-O-[4-[4-(N,N,N-trimethylammonio)butyl]phenyl]glycerol mesylate,

(2RS)-1-O-hexadecyl-2-dehydroxy-2-acetylamino-3-O-[4-[4-(N,N,N-trimethylammonio)butyl]phenyl]glycerol mesylate,

(2RS)-1-O-hexadecyl-2-dehydroxy-2-isobutoxycarbonylamino-3-O-[3-[4-(N,N-dimethylamino)phenyl]propyl]glycerol,

(2RS)-1-O-hexadecyl-2-dehydroxy-2-isobutoxycarbonylamino-3-O-[3-[4-(N,N,N-trimethylammonio)phenyl]propyl]glycerol iodide,

(2RS)-1-O-hexadecyl-2-dehydroxy-2-isobutoxycarbonylamino-3-O-[4-[3-(N,N-dimethylamino)propyl]benzyl]glycerol,

(2RS)-1-O-hexadecyl-2-dehydroxy-2-isobutoxycarbonylamino-3-O-[4-[3- (N,N,N-trimethylammonio)propyl]benzyl]glycerol iodide,

(2RS)-1-O-hexadecyl-2-dehydroxy-2-isobutoxycarbonylamino-3-O-[4-[4-(N,N-dimethylamino)butyl]benzyl]glycerol,

(2RS)-1-O-hexadecyl-2-dehydroxy-2-isobutoxycarbonylamino-3-O-[4-[4-(N,N,N-trimethylammonio)butyl]benzyl]glycerol iodide,

(2RS)-1-O-hexadecyl-2-dehydroxy-2-isobutoxycarbonylamino-3-O-[4-[3-(N,N-dimethylamino)propyl]phenyl]glycerol,

(2RS)-1-O-hexadecyl-2-dehydroxy-2-isobutoxycarbonylamino-3-O-[4-[3-(N,N,N-trimethylammonio)propyl]phenyl]glycerol iodide,

(2RS)-1-O-hexadecyl-2-dehydroxy-2-isobutoxycarbonylamino-3-O-[4-[4-(N,N-dimethylamino)butyl]phenyl]glycerol,

(2RS)-1-O-hexadecyl-2-dehydroxy-2-isobutoxycarbonylamino-3-O-[4-[4-(N,N,N-trimethylammonio)butyl]phenyl]glycerol iodide,

or

(2RS)-1-O-hexadecyl-2-dehydroxy-2-(N'-methylureido)-3-O-[4-[4-(N,N,N-trimethylammonio)butyl]phenyl]glycerol mesylate.

10. A process for the preparation of the compound of the general formula:

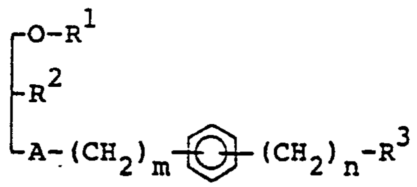

(I)

[wherein A represents an oxygen atom or a carbonyloxy group (wherein the carbonyl group should be attached to the group represented by $(CH_2)_m$ and the oxa group should be attached to the carbon atom at the 3rd position of the glycerol skelton), $R^1$ represents an alkyl group of 6 to 22 carbon atoms or an alkyl group of 2 to 5 carbon atoms being substituted by a phenyl group, $R^2$ represents a hydroxy group, an amino group, an azido group, an alkyl group of 1 to 20 carbon atom(s), an alkoxy group of 1 to 20 carbon atom(s) or a group of general formula:

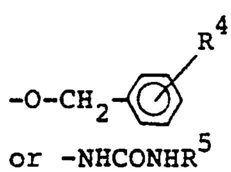

, $-OCOR^5$, $-OCONHR^5$, $-NHCOR^5$, $-NHCOOR^5$ or $-NHCONHR^5$ (wherein $R^4$ represents a hydrogen atom, a

halogen atom, an alkyl group of 1 to 4 carbon atom(s) or an alkoxy group of 1 to 4 carbon atom(s) and $R^5$ represents an alkyl group of 1 to 10 carbon atom(s) or a phenyl group), $R^3$ represents a group of general formula: $-N(R^6)_2$ or $-N^+(R^6)_3.Y^-$ (wherein each $R^6$ represents an alkyl group of 1 to 8 carbon atom(s) and which may be same or different to each other, or represents a heterocyclic ring of 4 to 10 ring members combining with 2 or 3 of $R^6$ and the nitrogen atom and if another $R^6$ not related to the heterocyclic ring exist, the $R^6$ represents an alkyl group of 1 to 8 carbon atom(s), and $Y^-$ represents an anion of acid.), m and n represents zero or an integer of from 1 to 6, respectively, and total number of m plus n should be more than 1. With the proviso that when A represents a carbonyloxy group, $R^2$ does not represent an amino group.]
which comprises:
(i) reaction of a compound of general formula:

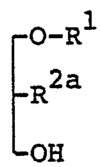

(II)

[wherein $R^{2a}$ represents a hydroxy group, an azido group , an alkyl group of 1 to 20 carbon atom(s), an alkoxy group of 1 to 20 carbon atom(s) or a group of general formula:

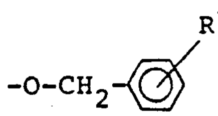 , $-NHCOR^5$, $-NHCOOR^5$ or $-NHCONHR^5$ (wherein $R^4$ and $R^5$ are as defined hereinbefore.) and $R^1$ is as defined hereinbefore.]

with a compound of general formula:

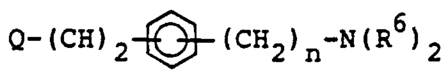 (III)

[wherein Q represents a halogen atom or an alkylsulphonyloxy group or an arylsulphonyloxy group being substituted or unsubstituted and the other symbols are as defined hereinbefore.]

and further subjecting the obtained compound to alkylation, if desired, and further subjecting to a method for salt-exchange, if desired;

(ii) removal of a boc group of a compound of general formula:

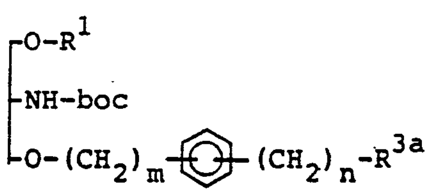 (V)

[wherein boc represents a tert-butylcarbonyl group and $R^{3a}$ represents a group of general formula: $-N(R^6)_2$ or $-N^+(R^6)_3.X^-$ (wherein $R^6$ is as defined hereinbefore and X represents a halogen atom.) and the other symbols are as defined hereinbefore.]

and further subjecting to a method for salt-exchange, if desired;

(iii) reaction of a compound of general formula:

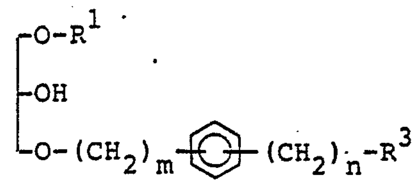

(Ia-3)

/wherein all the symbols are as defined hereinbefore./
with an acid of general formula:

$$HOOC-R^5 \quad (VI)$$

/wherein $R^5$ is as difined hereinbefore./
or an active derivative thereof (i.e. esterification)
or an isocyanate of general formula:

$$O=C=N-R^5 \quad (VII)$$

/wherein $R^5$ is as defined hereinbefore./
or
(iv) esterification of a compound of general formula:

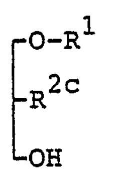

(VIII)

/wherein $R^{2c}$ represents a hydroxy group, an azido group, an alkyl group of 1 to 20 carbon atom(s), an alkoxy group of 1 to

20 carbon atom(s) or a group of general formula:

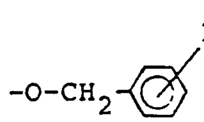 , $-OCOR^5$, $-OCONHR^5$, $-NHCOR^5$, $-NHCOOR^5$ or $-NHCONHR^5$ (wherein $R^4$ and $R^5$ are as defined hereinbefore.) and $R^1$ is as defined hereinbefore.]
with an acid of general formula:

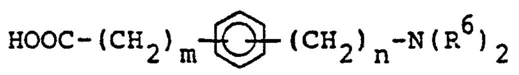 (X)

[wherein all the symbols are as defined hereinbefore.]
and further subjecting the obtained compound to alkylation, if desired, and further subjecting to a method for salt-exchange, if desired.

11. A process for the preparation of compounds of general formula:

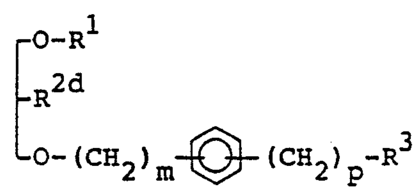 (Ie)

[wherein $R^{2d}$ represents a group of general formula:

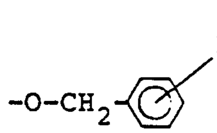 , $-NHCOR^5$, $-NHCOOR^5$ or $-NHCONHR^5$ (wherein $R^4$ and $R^5$ are as defined in claim 10.), p represents an integer of from 1 to 6 and the other symbols are as defined in claim 10.]

which comprises:

reaction of a compound of general formula:

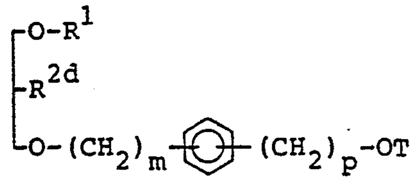

(X)

[wherein T represents an alkylsulphonyl group or an arylsulphonyl group being substituted or unsubstituted, and the other symbols are as defined hereinbefore.]

with a compound of general formula:

$$NH(R^6)_2 \qquad (XI)$$

or

$$N(R^6)_3 \qquad (XII)$$

[wherein $R^6$ is as defined in claim 10.]

and further subjecting the obtained compound to alkylation, if desired, and further subjecting to a method for salt-exchange, if desired.

12. A process for the preparation of compound of general formula:

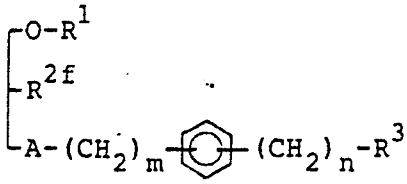

(If)

/wherein (i) when A represents an oxygen atom, $R^{2f}$ represents an amino group, an azido group or a group of general formula:
$-NHCOR^5$, $-NHCOOR^5$ or $-NHCONHR^5$ (wherein $R^5$ is as defined in claim 10.), (ii) when A represents a carbonyloxy group, $R^{2f}$ represents a group of general formula: $-OCOR^5$ or $-OCONHR^5$ (wherein $R^5$ is as defined hereinbefore.) and the other symbols are as defined in claim 10./
which comprises:

(i) reaction of a compound of general formula:

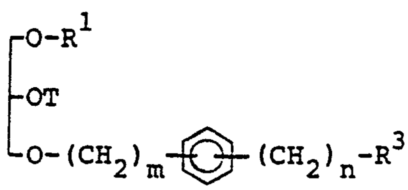

(LI)

/wherein T is as defined in claim 11, and the other symbols are as defined hereinbefore./
with sodium azide;

(ii) reduction of a compound of general formula:

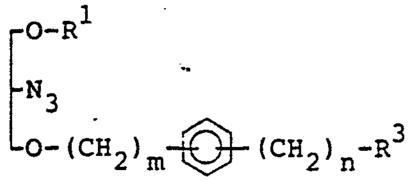

(If-1)

[wherein all the symbols are as defined hereinbefore.];

(iii) reaction of a compound of general formula:

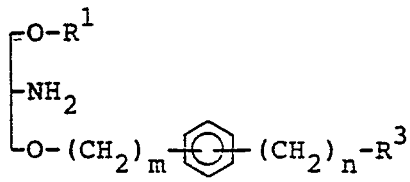

(If-2)

[wherein all the symbols are as defined hereinbefore.]
with a compound of general formula:

$$X^1\text{-}COR^5 \qquad (XLVI)$$

or

$$R^5\text{-}COO\text{-}CO\text{-}R^5 \qquad (XLVII)$$

[wherein $X^1$ represents a halogen atom and $R^5$ is as defined hereinbefore.],

(iv) reaction of a compound of the general formula (If-2) with a compound of general formula:

$$X^2\text{-}COOR^5 \qquad \text{(XLVIII)}$$

[wherein $X^2$ represents a halogen atom, and $R^5$ is as defined hereinbefore.];

(v) reaction of a compound of the general formula (If-2) with a compound of general formula:

$$O{=}C{=}N\text{-}R^5 \qquad \text{(VII)}$$

[wherein $R^5$ is as defined hereinbefore.];

(vi) esterification of a compound of the general formula:

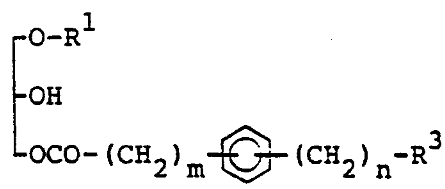

(Id-1)

[wherein all the symbols are as defined hereinbefore.] with an acid of general formula:

$$HOOC\text{-}R^5 \qquad \text{(VI)}$$

[wherein $R^5$ is as defined hereinbefore.] or an active derivative thereof

or

(vii) reaction of a compound of the general formula (Id-1) with a compound of the general formula (VII).

13. A process for the preparation of compounds of general formula:

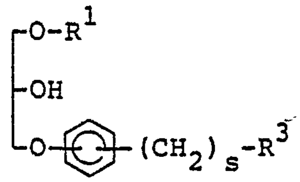

(Ig)

/wherein, s represents an integer of from 2 to 6, and $R^1$ and $R^3$ are as defined in claim 10./
which comprises removal of a benzyl group and converting a vinylene group into an ethylene group of a compound of general formula:

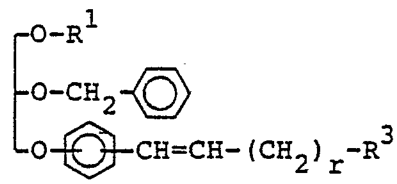

(LII)

/wherein r represents zero or an integer of from 1 to 4, and the other symbols are as defined hereinbefore./
at the same time, by subjecting to reduction reaction.

14. A platelet aggregation inhibiting, anti-asthmatic, anti-allergic, anti-inflammatory, hypotensive or anti-tumour pharmaceutical composition, which contains, as active ingredient(s), compound(s) of the general formula (I) wherein various symbols are defined in claim 1, or acid additional salt(s) thereof with a pharmaceutical carrier of coating.

15. A method of inhibiting pletelet aggregation, inflammation, hypotention and tumour which comprises the oral or parenteral administration of an effective amount of a compound as claimed in claim 1 wherein various symbols are as defined in claim 1, or an acid additional salt thereof.

European Patent Office

**PARTIAL EUROPEAN SEARCH REPORT**
which under Rule 45 of the European Patent Convention shall be considered, for the purposes of subsequent proceedings, as the European search report

0145303
Application number
EP 84307817.1

## DOCUMENTS CONSIDERED TO BE RELEVANT

| Category | Citation of document with indication, where appropriate, of relevant passages | Relevant to claim |
| --- | --- | --- |
| D,P,A | EP - A2 - 0 109 255 (ONO PHARMACEUTICAL CO., LTD.) * Claims 1,7-10 * -- | 1-8, 10-12, 14 |
| D,P A | EP - A2 - 0 094 586 (ONO PHARMACEUTICAL CO., LTD.) * Claims 1,2,4-6,22,24,25,28, 31 * -- | 1-8, 10-12, 14 |
| D,A | DE - A1 - 2 835 369 (PFIZER INC.) * Claims 1-4,6,22,29; pages 17-23 * -- | 1-4,6, 8,10-12,14 |
| A | GB - A - 2 021 580 (PFIZER INC.) * Claims 1,8; page 2, lines 30-52 * ---- | 1,14 |

CLASSIFICATION OF THE APPLICATION (Int. Cl.[3])

C 07 C 125/063
C 07 C 93/00
C 07 C 117/00
C 07 C 127/15
A 61 K 31/13
A 61 K 31/16
A 61 K 31/17
A 61 K 31/22
A 61 K 31/27

TECHNICAL FIELDS SEARCHED (Int. Cl.[3])

C 07 C 125/00
C 07 C 93/00
C 07 C 91/00
C 07 C 117/00
C 07 C 127/00
C 07 C 43/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-14
Claims searched incompletely: –
Claims not searched: 15
Reason for the limitation of the search: Method for treatment of the human or animal body by therapy (Art. 52(4) EPC)

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
| --- | --- | --- |
| VIENNA | 13-02-1985 | HERING |